# EUROPÄISCHE PATENTSCHRIFT

(11) **EP 0 409 125 B1**
(45) Veröffentlichungstag und Bekanntmachung des Hinweises auf die Patenterteilung: **03.01.1996**
(21) Anmeldenummer: 90113546.7
(22) Anmeldetag: 15.07.1990
(51) Int. Cl.: C07D 487/14

(54) **Salze von N5, N10 - Methylen-5,6,7,8-tetrahydrofolsäure**
Salts of N5, N10-methylene-5,6,7,8-tetrahydrofolic acid
Sels de l'acide N5, N10-méthylène-5,6,7,8-tétrahydrofolique

(30) Priorität: 21.07.1989 CH 2736/89; 12.04.1990 CH 1256/90
(43) Veröffentlichungstag der Anmeldung: 23.01.1991
(73) Patentinhaber: Cerbios-Pharma S.A., CH-6917 Barbengo (CH)
(72) Erfinder: Marazza, Fabrizio, CH-6921 Vico Morcote (CH); Melera, Attilio, CH-6926 Montagnola (CH); Viterbo, René, F-75007 Paris (FR); Toderi, Nando, CH-6982 Agno (CH)
(74) Vertreter: Zink, Markus, Dr.

(56) Entgegenhaltungen:
- EP-A- 0 293 029
- FR-A- 2 081 399
- FR-A- 2 137 186
- GB-A- 1 572 138
- BIOCHEMISTRY, Band 18, Nr. 24, 1979, Seiten 5527-5530, American Chemical Society; M. POE et al.: "5,10-Methylene-5,6,7,8-tetrahydrofolate. Conformation of the tetrahydropyrazine and imidazolidine rings"
- The Journal of Biological Chemistry 238, (1963), 1498-1500
- Biochemical and Biophysical research Communications 14,(1964),523-526
- Cancer Research 49,(1989), 2592-2596
- The Journal of Biological Chemistry 238, +1963), 3075 -3079
- Chem .Abstr. 98, N 215624r
- Cancer 63, (1963), 995 - 1007
- Royal Society of Medicines Services Int. Congress and Symposium Series N 158, publ. by Royal Society of Medicine Services Ltd.,12. April 1989

## Beschreibung

Die vorliegende Erfindung betrifft Salze von N⁵,N¹⁰-Methylen-5,6,7,8-tetrahydrofolsäure, Verfahren zu deren Herstellung sowie deren Verwendung zur Herstellung eines Arzneimittels für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen.

### Hintergrund der Erfindung

N⁵,N¹⁰-Methylen-5,6,7,8-tetrahydrofolsäure der Formel II (abgekürzt mit CH₂-THF) ist die chemische Spezies, welche als Co-Faktor bei der Inhibierung des Enzyms Thymidilatsynthetase (abgekürzt mit TS) mit 5-Fluorouracil (abgekürzt mit 5-FU) und/oder einer der Vorstufen (prodrug), wie etwa Floxuridine, Tegafur, wirkt. 5-FU oder eine der Vorstufen werden metabolisch in 5F-Desoxyuridinemonophosphat (abgekürzt mit 5F-dUMP) transformiert, welches kovalent und irreversibel an TS in der Gegenwart von CH₂-THF gebunden ist, wobei ein ternärer Komplex gebildet wird, welcher als 5F-dUMP/TS/CH₂-THF beschrieben wird.

5-FU und seine Vorstufen werden in grossem Mass verwendet als chemotherapeutische Mittel bei der Behandlung von Kolon, rektalem und pankreatischem Karzinom, wie auch bei weiteren Formen von Krebs, wie etwa Brustkrebs und Magenkrebs. Um die Cytotoxizität für die Tumorzellen zu erhöhen, muss die höchste Inhibitionsrate von TS erreicht werden. Dies erfordert eine geeignete Versorgung jener Zellen mit 5-FU (oder eine seiner Vorstufen) und mit dem Co-Faktor CH₂-THF. Währenddem 5-FU (oder seine Vorstufen) exogene Verbindungen sind, welche einem Patienten in jeder gewünschten Menge verabreicht werden können, hat CH₂-THF normalerweise seinen Ursprung in der endogenen Folat-Zusammenlegung via eine Anzahl von metabolischen Umwandlungen. Jedoch kann die auf diesem Weg gebildete Menge an CH₂-THF nicht genügend sein für eine effiziente und komplette Inhibierung von TS.

Es hat sich daher zur Gewohnheit entwickelt, klinische Behandlungsprotokolle zu verwenden, in welchen Calcium-folinat (abgekürzt mit CaF), ein metabolischer Vorläufer von CH₂-THF, in hohen Dosen zusammen mit 5-FU einem Patienten zur Verfügung gestellt werden. Siehe R.J. DeLap, The Yale Journal of Biology and Medicine, 61, 23 (1988). Diese hohen Dosen an CaF resultieren in Nebeneffekten, wie etwa Diarrhöe und Stomatitis; siehe G. Marini et al. Oncology 44, 336 (1987). Die Versorgung eines Patienten mit adäquaten Mengen an CH₂-THF, d.h. die den ternären Komplex bildende Species selbst, konnte bis jetzt nicht erreicht werden, weil kein grosstechnisches Verfahren zur Herstellung einer genügend reinen, gut wasserlöslichen und stabilen Verbindung zur Verfügung stand.

### Stand der Technik

Die Herstellung in kleinem Massstab von CH₂-THF als freie Säure ist von verschiedenen Autoren beschrieben worden; siehe R.L. Blakley, Biochem. Journal, 72, 707 (1959); M.J. Osborn, P.T. Talbert, F.M. Huennekens, J.Am. Chem. Soc. 82, 4921 (1960); R.G. Kallen, W.P. Jenks, J.Biol. Chem. 241, 5851 (1966); P.R. Farina, L.J. Farina, S.J. Benkovic, J.Am. Chem. Soc. 95, 5409 (1973); R.P. Leary, Y. Gaumont, R.L. Kisliuk, Biochem. Biophys. Res. Comm. 56, 484 (1974); R.L. Kisliuk, D. Strumpf, Y. Gaumont, R.P. Leary, L. Palnte, J. Med. Chem. 20, 1531 (1977); M. Poe, L.M. Jackman, S.T. Benkovic, Biochemistry 18, 5527 (1979) und R. Kalbermatten, W. Staedeli, J.H. Bieri, M. Viscontini, Helv. Chim. Acta 64, 2627 (1981).

In Cancer Research, Vol. 49, (1989), Seiten 2592 - 2596 wird N(5), N(10)-Methylen-5,6,7,8-tetrahydrofolsäure (abgekürzt mit CH₂-THF) in kleinsten Mengen als Referenzsubstanz für analytische Zwecke verwendet.

Als Salze von CH₂-THF werden die entsprechenden Salze von Trisbase verwendet.

C. Zarow, A.M. Pellino, P.V. Daneberg, Prep. Biochem. 12, 281 (1983) beschreiben ein Herstellungsverfahren "in grossem Massstab", welches eine enzymatische Reduktionsstufe umfasst.

In allen Fällen konnte jedoch das Produkt nicht anders isoliert werden als in lyophilisierter Form oder als ein amorphes Pulver (siehe C.M. Tatum Jr., P.A. Benkovic, S.T. Benkovic, R. Potts, Biochemistry 16, 1903 [1977]) und in einer Reinheit von nicht höher als 80 %, was diese Produkte unbrauchbar für die therapeutische Verwendung bei Menschen macht.

Zwei Syntheseverfahren sind beschrieben worden, um CH₂-THF als freie Säure zu erhalten.

### Erstes Verfahren

5,6,7,8-Tetrahydrofolsäure der Formel IV 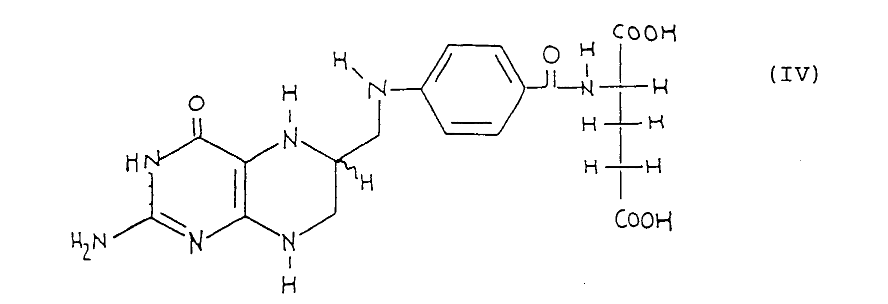 wird mit einem Ueberschuss an Formaldehyd in wässriger Lösung bei einem pH-Wert von 7 umgesetzt. Siehe dazu M. Poe, L.J. Jackman, S.T. Benkovic, Biochemistry 18, 5527 (1979); R. Kalbermatten, W. Staedeli, J.H. Bieri, M. Viscontini, Helv. Chim. Acta 64, 2627 (1981); C. Zarow, A.M. Pellino, P.V. Danenberg, Prep. Biochem. 12, 381 (1983). Dieses Verfahren ist nur geeignet für die Herstellung von Milligramm-Mengen an freier Säure der Formel II 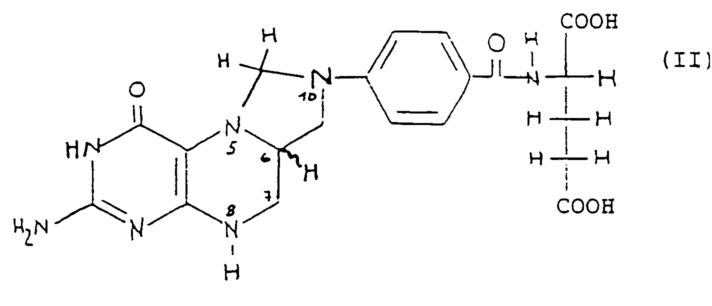 und führt, wegen der inhärenten Instabilität von CH₂-THF in wässriger Lösung mit pH 7, zu einem Produkt mit geringer Reinheit (weniger als 80 %); siehe R.G. Kallen, W.P. Jenks, J. Biol. Chem. 241, 5851 (1966).

### Zweites Verfahren

N⁵,N¹⁰-Methenyl-5,6,7,8-tetrahydrofolsäure in Form des Chlorides der Formel V 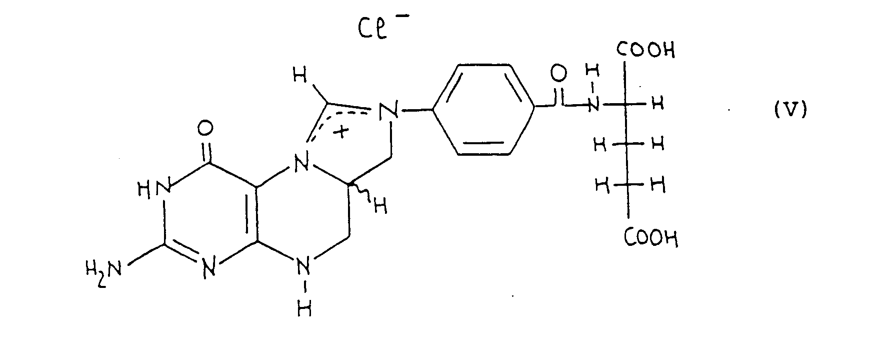 wird in einem wasserfreien Lösungsmittel mit NaBH₄ reduziert. CH₂-THF als freie Säure der oben genannten Formel II wird als ein amorpher Festkörper mit einer Reinheit von 75 bis 80 % isoliert; siehe dazu P.R. Farina, L.J. Farina, S.J. Benkovic, J. Am. Chem. Soc. 95, 5409 (1973); C.M. Tatum Jr., P.A. Benkovic, S.T. Benkovic, R. Potts, Biochemistry 16, 1903 (1977). CH₂-THF in Form der freien Säure ist praktisch unlöslich in Wasser, und ist als solche ungeeignet für parenterale pharmazeutische Präparate.

Völlig überraschend wurden nun Verfahren gefunden, welche für die grosstechnische Produktion geeignet sind, zur Herstellung von neuen, stabilen und therapeutisch annehmbaren, wasserlöslichen Salzen von CH₂-THF, welche genügend rein sind, um einem Patienten verabreicht zu werden.

Die vorliegende Erfindung ist durch die Merkmale in den unabhängigen Ansprüchen charakterisiert. Bevorzugte Ausführungsformen sind in den abhängigen Ansprüchen definiert.

### Beschreibung der Erfindung

In der Tabelle I sind einige bevorzugte erfindungsgemässe Verbindungen zusammengefasst. 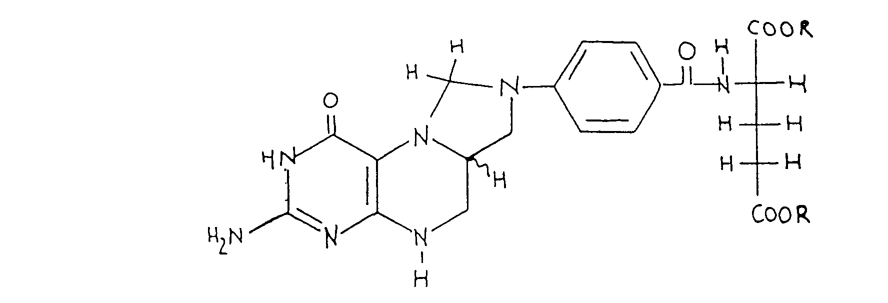
3a R = H (bekannt)
3b R = Na⁺,Pyridinium
3c R = [HO(CH₂)₂NH₃]⁺
3d R = {[HO(CH₂)₂]₃NH}⁺
3e R = [HO(CH₂)₂NH(CH₃)₂]⁺
3f R = [(CH₃)₃CNH₃]⁺
3g R = [(L)-Lysin ]⁺
3h R = [(L)-Arginin ]⁺
3i R = 1/2 Ca⁺
3k R = 1/2 Mg⁺

In der Tabelle II wird die Herstellung von einigen bevorzugten erfindungsgemässen Verbindungen schematisch dargestellt.

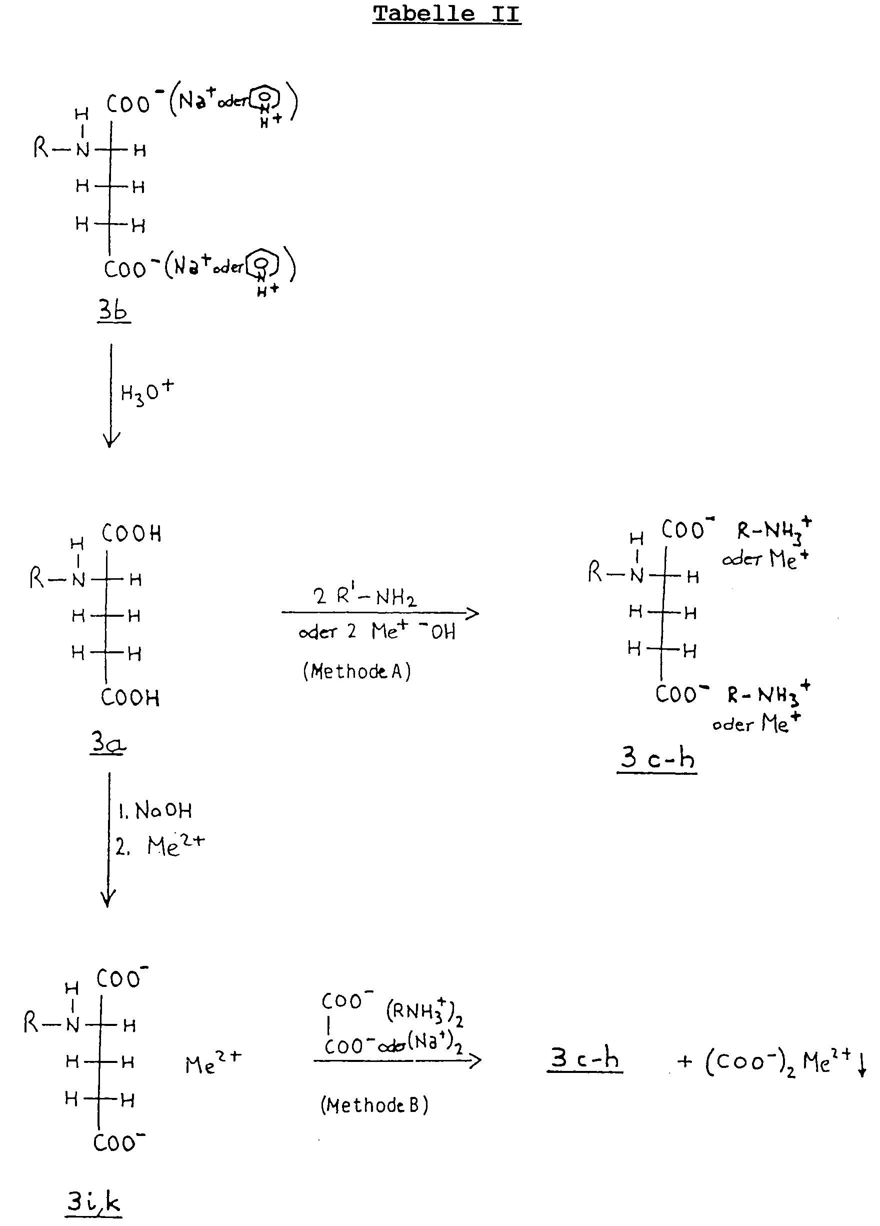

R= N⁵,N¹⁰-Methylen-5,6,7,8-tetrahydropteroyl

Die verschiedenen erfindungemässen Salze können entweder direkt aus der Verbindung der Formel 3a (Methode A) oder mittels der Reaktion eines Erdalkalimetallsalzes, beispielsweise das Kalziumsalz der Formel 3i, mit dem Oxalsäuresalz des entsprechenden Kations oder Amins (Methode B) hergestellt werden.

Die HPLC Reinheit des auf diesem Wege erhaltenen CH₂-THF Salzes variiert zwischen 83,8 und 92,4 %; die Haupt-"Verunreinigung" dieser Herstellung besteht aus der gut bekannten und therapeutisch wertvollen (6RS)-N⁵-Methyl-5,6,7,8-tetrahydrofolsäure, so dass die gesamte Menge an reduzierten Folaten bei der Herstellung im Durchschnitt 95 % beträgt.

### Methode A

Die Verbindung der Formel 3a wurde hergestellt mittels der Umsetzung der Verbindung der Formel V in einem Lösungsmittel, wie etwa DMSO (Dimethylsulfoxid) in der Gegenwart einer Base, wie etwa Pyridin, mit der jeweiligen Menge an NaBH₄, wobei man die Verbindung der Formel 3b erhält, welche weiter gelöst wird in einer kalten KOH-Lösung; die Verbindung der Formel 3a wird erhalten mittels Ansäuern.

Die Verbindung der Formel 3a wird nacheinander umgesetzt mit einer pharmazeutisch annehmbaren Base, wie etwa Ethanolamin, Triethanolamin, 2-Dimethylaminoethanol, tert.-Butylamin, oder mit Aminocarbonsäuren, wie etwa Lysin, Arginin, wobei wasserlösliche Salze gebildet werden. Die Verbindung der Formel 3a kann auch mit den Kationen Ca⁺, Mg⁺, umgesetzt werden, wobei die entsprechenden wasserlöslichen Salze gebildet werden, welche isoliert werden mittels Ausfällen aus der wässrigen Lösung mittels der Hinzugabe eines organischen Lösungsmittels, wie etwa Ethanol, Aceton. Siehe Tabelle II.

### Methode B

Die Erdalkalimetallsalze, beispielsweise die Verbindung der Formel 3i, welche mittels der Methode A erhalten wurden, wurden mit dem Oxalsäuresalz des ausgewählten Amins oder der entsprechenden Aminosäure oder des entsprechenden Kations umgesetzt. Bedingt durch die Ausfällung des wasserunlöslichen Erdalkalimetalloxalates bleibt das entsprechend abgewandelte Salz in Lösung und wird erst ausgefällt mittels der Hinzugabe eines organischen wasserlöslichen Lösungsmittels, wie etwa Ethanol, Aceton. Siehe Tabelle II.

### Methode C

Völlig überraschend wurde nun noch ein weiteres, drittes Verfahren, welches für die grosstechnische Produktion geeignet ist, zur Herstellung von Erdalkalimetallsalzen von N⁵,N¹⁰-Methylen-5,6,7,8-tetrahydrofolsäure gefunden.

Mit dem dritten erfindungsgemässen Verfahren werden die Ca⁺ und Mg⁺ Salze in grosser Ausbeute und in grosser Reinheit erhalten. Zudem wird in diesem erfindungsgemässen Verfahren in wässrigem Medium gearbeitet, was die Isolierung der gewünschten Erdalkalimetallsalze erheblich vereinfacht.

Bei diesem erfindungsgemässen Verfahren legt man eine wässrige Lösung eines wasserlöslichen Salzes der 5,6,7,8-Tetrahydrofolsäure vor und bringt diese dann mit Formaldehyd zur Reaktion. Anschliessend wird eine wässrige Lösung eines Ca⁺ oder Mg⁺ Salzes hinzugegeben, wobei das entsprechende Ca⁺ oder Mg⁺ Salz ausfällt.

Die 5,6,7,8-Tetrahydrofolsäure kann beispielsweise gemäss den Angaben von E. Khalifa, A.N. Ganguly, J.H. Bieri und M. Viscontini, Helv. Ch. Acta (1980), 63, 2554 hergestellt werden.

Die erfindungsgemässen Salze können verwendet werden zur Herstellung eines Arzneimittels für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen. Ein solches Arzneimittel enthält gewöhnlich wenigstens ein aktives Salz in einer Menge von 1 mg bis 500 mg, insbesondere von 5 mg bis 150 mg, pro Dosiseinheit. Das Arzneimittel liegt vorzugsweise in Form eines parenteralen und/oder oralen pharmazeutischen Präparates vor.

Die nachfolgenden Beispiele dienen der Illustration der Erfindung. Klinische Resultate und therapeutische Anwendungen dieser Verbindungen werden ebenfalls beschrieben.

### Beispiele

### Beispiel 1

Herstellung der (6RS)-N⁵,N¹⁰-Methylen-5,6,7,8-tetrahydrofolsäure der Formel 3a.

Zu einer Lösung von 24,6 g der Verbindung der Formel V (hergestellt gemäss C. Temple, R.D. Elliot, J.D. Rose, J.A. Montgomery, J. Med. Chem. 22, 731 [1979]) in einem Gemisch aus 300 ml trockenem DMSO und 100 ml trockenem Pyridin, gerührt unter Sauerstoffausschluss bei Raumtemperatur, wurde tropfenweise eine Lösung von 2,0 g NaBH₄ in 100 ml DMSO während einer Stunde hinzugegeben. Dieses Gemisch wurde anschliessend mit zwei Liter Aceton während einer Zeitspanne von 20 Minuten verdünnt. Nach einem weiteren Rühren des Gemisches während einer Stunde bei einer Temperatur von +4° C wurde das gefällte gemischte Salz der Formel 3b mittels Filtration gesammelt und dreimal mit frischem Aceton gewaschen. Nach dem Trocknen unter reduziertem Druck wurden 16,17 g rohe Verbindung der Formel 3b isoliert.

Die HPLC Analyse zeigte, dass dieses Produkt 87,1 % rein war.

Das oben genannte Produkt wurde zu 420 ml entgastem Wasser hinzugegeben, und unter Sauerstoffausschluss bei einer Temperatur von +4° C gerührt. 0,5 M kalte KOH wurde anschliessend tropfenweise zur gerührten Suspension hinzugegeben, bis eine vollständige Auflösung beobachtet wurde. Der pH-Wert der so erhaltenen Lösung betrug 7,5 und die freie Säure der Formel 3a wurde ausgefällt mittels der tropfenweisen Hinzugabe von kalter 1N HCl, bis der pH-Wert der überstehenden Lösung einen Wert von 3,95 erreichte. Nach einem weiteren Rühren bei +4° C während 10 Minuten wurde die freie Säure der Formel 3a mittels Filtration gesammelt, einmal mit kaltem Wasser gewaschen sowie einmal mit Aceton und einmal mit peroxidfreiem Diethyläther gewaschen.

Nach dem Trocknen unter reduziertem Druck wurden 11,36 g der amorphen Verbindung der Formel 3a isoliert.

HPLC-Analyse: 86,5 % Reinheit / 7,6 % N⁵-Methyl-THF.

Dieses Produkt wurde nicht weiter gereinigt, sondern in verschiedene Salze, wie weiter unten beschrieben, übergeführt.

### Beispiel 2

Herstellung des Ethanolaminsalzes von (6RS)-N⁵,N¹⁰-Methylen-5,6,7,8-tetrahydrofolsäure der Formel 3c (Methode A).

Zu einer Lösung von 11,36 g der Verbindung der Formel 3a, gelöst in 170 ml trockenem DMSO und gerührt bei Raumtemperatur unter Sauerstoffausschluss, wurde eine Lösung von 3,0 ml Ethanolamin (2 Equivalente) in 50 ml DMSO tropfenweise hinzugegeben. Das Gemisch wurde anschliessend mittels Filtration von einer leichten Trübung befreit und langsam zu 600 ml gerührtem Ethylacetat hinzugegeben. Nach einem weiteren Rühren während 20 Minuten bei Raumtemperatur wurde das ausgefallene Produkt mittels Filtration gesammelt und zweimal mit Ethylacetat und einmal mit Diethylether gewaschen. Nach dem Trocknen unter reduziertem Druck wurden 14,22 g der Verbindung der Formel 3c isoliert.

HPLC-Analyse: 83,8 % Reinheit / 7,1 % N⁵-Methyl-THF.

UV (c = 20 mg/l in 0,1 M Phosphat-Puffer bei pH 7,3): λₘₐₓ = 293 nm (ε = 43000) λₘᵢₙ = 247 nm Aₘₐₓ / Aₘᵢₙ = 3,9.

[α]_{D} = + 10,4° (c = 1 in 0,1 M Phosphat-Puffer bei pH 7,3).

NMR (220 MHz / in DMSO-Pyridin 9:1) : 2 charakteristische Doublets (J = 4 Hz) bei 5,05 ppM (1H) / 3,85 ppM (1H) für die C-11 Methylenprotonen.

### Beispiel 3

Herstellung des Triethanolaminsalzes von (6RS)-N⁵,N¹⁰-Methylen-5,6,7,8-tetrahydrofolsäure der Formel 3d (Methode A).

Zu einer Lösung von 4,35 g der Verbindung der Formel 3a in 50 ml trockenem DMSO, gerührt bei Raumtemperatur unter Argon, wurde tropfenweise eine Lösung von 2,84 g Triethanolamin (2 Equivalente) in 14 ml DMSO hinzugegeben. Nach dem Abfiltrieren einer kleinen Menge an weissem Niederschlag wurde die Lösung tropfenweise zu einem gerührten Gemisch von 220 ml Ethanol und 110 ml Diethylether hinzugegeben.

Die Suspension wurde während weiteren 30 Minuten bei einer Temperatur von +4° C gerührt. Der Festkörper wurde mittels Filtration gesammelt, einmal mit Ethanol/Diethylether 2:1, und einmal mit Diethylether gewaschen, und unter reduziertem Druck getrocknet, wobei man 4,88 g der Verbindung der Formel 3d erhielt.

HPLC-Analyse: 90,3 % Reinheit / 5,8 % N⁵-Methyl-THF.

UV (c = 20 mg/l in 0,1 M Phosphat-Puffer bei pH 7,3):

λₘₐₓ = 295 nm (ε = 37800) λₘᵢₙ = 246 nm Aₘₐₓ/Aₘᵢₙ = 4,9.

[α]_{D}= +11,7° (c=1 in 0,1 M Phosphat-Puffer bei pH 7,3).

NMR (220 MHz / in D₂O/NaOD) : 2 charakteristische Doublets (J = 4 Hz) bei 5,03 ppM (1H) / 3,90 ppm (1H) für die C-ll Methylenprotonen.

### Beispiel 4

Herstellung des 2-Dimethylaminoethanolsalzes von (6RS)-N⁵,N¹⁰-Methylen-5,6,7,8-tetrahydrofolsäure der Formel 3e (Methode A).

Zu einer Lösung von 4,0 g der Verbindung der Formel 3a in 46 ml trockenem DMSO, gerührt bei Raumtemperatur unter Sauerstoffausschluss, wurde tropfenweise eine Lösung von 1,76 ml 2-Dimethylaminoethanol (2 Equivalente) in 13 ml DMSO hinzugegeben. Nach dem Abfiltrieren einer kleinen Menge an Niederschlag wurde die Lösung tropfenweise zu einem gerührten Gemisch aus 200 ml Ethanol und 100 ml Diethylether bei einer Temperatur von +4°C hinzugegeben. Nach dem Rühren während weiteren 30 Minuten bei der gleichen Temperatur wurde der Festkörper mittels Filtration gesammelt, und einmal mit Ethanol-Diethylether 2:1 und einmal mit Diethylether gewaschen. Nach dem Trocknen unter reduziertem Druck wurden 3,93 g der Verbindung der Formel 3e isoliert.

HPLC-Analyse: 91 % Reinheit / 5,7 % N⁵-Methyl-THF.

UV (c = 20 mg/l in 0,1 M Phosphat-Puffer bei pH 7,3):

λₘₐₓ = 295 nm (ε = 31200) λₘᵢₙ = 246 nm Aₘₐₓ/Aₘᵢₙ = 4,5.

[α]_{D}= +12,1° (c=1 in 0,1 M Phosphat-Puffer bei pH 7,3).

NMR (220 MHz / in D₂O/NaOD) : 2 charakteristische Doublets (J = 4 Hz) bei 5,03 ppM (1H) / 3,90 ppm (1H) für die C-11 Methylenprotonen.

### Beispiel 5

Herstellung des tert.-Butylaminsalzes von (6RS)-N⁵,N¹⁰-Methylen-5,6,7,8-tetrahydrofolsäure der Formel 3f (Methode A).

Zu einer Lösung von 4,3 g der Verbindung der Formel 3a in 60 ml trockenem DMSO, gerührt bei Raumtemperatur unter Argon, wurde tropfenweise eine Lösung von 1,97 ml tert.-Butylamin (2 Equivalente) in 17 ml DMSO hinzugegeben. Nach wenigen Minuten des Rührens wurde ein dicker Niederschlag gebildet. Das Gemisch wurde weiter bei einer Temperatur von +18°C während einer Stunde gerührt. Das Produkt wurde isoliert mittels wiederholter Zentrifugation bei 3500 U/Min. (rpm), wobei das Produkt vorgängig in einem Gemisch von 100 ml Ethanol und 50 ml Diethylether und danach in reinem Diethylether suspendiert wurde.

Nach dem Trocknen unter reduziertem Druck wurden 4,63 g der Verbindung der Formel 3f isoliert.

HPLC-Analyse: 89 % Reinheit / 7,1 % N⁵-Methyl-THF.

UV (c = 20 mg/l in 0,1 M Phosphat-Puffer bei pH 7,3):

λₘₐₓ = 295 nm (ε = 32700) λₘᵢₙ = 246 nm Aₘₐₓ/Aₘᵢₙ = 4,5.

[α]_{D}= +11,0° (c=1 in 0,1 M Phosphat-Puffer bei pH 7,3).

NMR (220 MHz / in D₂O/NaOD) : 2 charakteristische Doublets (J = 4 Hz) bei 5,03 ppM (1H) / 3,90 ppm (1H) für die C-11 Methylenprotonen.

### Beispiel 6

Herstellung des (L)-Lysinsalzes von (6RS)-N⁵,N¹⁰-Methylen-5,6,7,8-tetrahydrofolsäure der Formel 3g (Methode A).

Zu einer Lösung von 4,05 g der Verbindung der Formel 3a in 55 ml trockenem DMSO, gerührt bei Raumtemperatur unter Argon, wurde tropfenweise eine Lösung von 2,60 g (L)-Lysin (2 Equivalente) in 15 ml DMSO hinzugegeben. Nach dem Rühren während einigen Minuten wurde ein dicker Niederschlag gebildet. Das Gemisch wurde weiterhin bei einer Temperatur von +18°C während einer Stunde gerührt. Das Produkt wurde isoliert durch wiederholte Zentrifugation bei 3500 rpm, wobei das Produkt vorgängig in einem Gemisch von 100 ml Ethanol und 50 ml Diethylether und anschliessend in reinem Diethylether suspendiert wurde.

Nach dem Trocknen unter reduziertem Druck wurden 5,03 g der Verbindung der Formel 3g isoliert.

HPLC-Analyse: 90 % Reinheit / 6,1 % N⁵-Methyl-THF.

UV (c = 20 mg/l in 0,1 M Phosphat-Puffer bei pH 7,3):

λₘₐₓ = 295 nm (ε = 29500) λₘᵢₙ = 246 nm Aₘₐₓ/Aₘᵢₙ = 4,4.

[α]_{D}= +11,5° (c=1 in 0,1 M Phosphat-Puffer bei pH 7,3).

NMR (220 MHz / in D₂O/NaOD) : 2 charakteristische Doublets (J = 4 Hz) bei 5,03 ppM (1H) / 3,90 ppm (1H) für die C-11 Methylenprotonen.

### Beispiel 7

Herstellung des (L)-Argininsalzes von (6RS)-N⁵,N¹⁰-Methylen-5,6,7,8-tetrahydrofolsäure der Formel 3h (Methode A).

Das (L)-Argininsalz der Formel 3h wurde in analoger Art und Weise, wie in Beispiel 6 beschrieben, hergestellt.

### Beispiel 8

Herstellung des Calciumsalzes von (6RS)-N⁵,N¹⁰-Methylen-5,6,7,8-tetrahydrofolsäure der Formel 3i (Methode A).

1,0 g der rohen Verbindung der Formel 3b wurde in kleinen Mengen zu einem Gemisch aus 16 ml entgastem Wasser und einem Tropfen lN NaOH hinzugegeben, und heftig bei einer Temperatur von +4°C gerührt. Während der Hinzugabe wurde der pH-Wert des Gemisches bei etwa 9,0 mittels der gelegentlichen Hinzugabe von lN NaOH gehalten. Danach wurden 0,92 ml einer 2M Kalziumchloridlösung zum Gemisch hinzugegeben. Nach dem Rühren während 10 Minuten wurde die Lösung von einem unlöslichen Niederschlag mittels Filtration befreit. Schlussendlich wurde das Gemisch tropfenweise mit 20 ml Aceton unter Rühren bei einer Temperatur von +4°C verdünnt, wobei man einen gelblichen Niederschlag erhielt, welcher mittels Filtration isoliert wurde, und einmal mit absolutem Ethanol und einmal mit Aceton gewaschen wurde und unter reduziertem Druck getrocknet wurde. Auf diese Art und Weise wurden 673 mg des Kalziumsalzes der Formel 3i isoliert. HPLC-Analyse: 91 % Reinheit / 6% N⁵-Methyl-THF.

UV (c = 20 mg/l in 0,1 M Phosphat-Puffer bei pH 7,3): λₘₐₓ = 296 nm (ε = 28900) λₘᵢₙ = 245 nm Aₘₐₓ/Aₘᵢₙ = 3,68.

[α]_{D}= +12,6° (c = 1 in 0,01 N NaOH).

H₂O Gehalt (nach Karl Fischer): 8,9 %.

**Elementaranalyse für CH₂-THF.Ca x 3,5 H₂O**

| | Berechneter Wert | exp. Wert |
|---|---|---|
| %C | 43,02 | 43,27 |
| %H | 5,05 | 4,84 |
| %N | 17,56 | 17,57 |
| %Ca | 7,18 | 6,99. |

### Beispiel 9

Herstellung des Magnesiumsalzes von (6RS)-N⁵,N¹⁰-Methylen-5,6,7,8-tetrahydrofolsäure der Formel 3k (Methode A).

1,0 g der rohen Verbindung der Formel 3b wurden in kleinen Portionen zu einem Gemisch aus 16 ml entgastem Wasser und einem Tropfen lN NaOH hinzugegeben, und heftig bei einer Temperatur von +4°C gerührt. Während der Hinzugabe wurde der pH-Wert des Gemisches bei etwa 9,0 mittels der gelegentlichen Hinzugabe von lN NaOH gehalten. Anschliessend wurden 0,92 ml einer 2M Magnesiumchloridlösung zum Gemisch hinzugegeben. Nach dem Rühren während 10 Minuten wurde die Lösung von einem kleinen unlöslichen Niederschlag mittels Filtration befreit. Das Gemisch wurde tropfenweise mit 20 ml Aceton verdünnt, wobei das Gemisch bei einer Temperatur von +4°C gerührt wurde. Man erhielt einen gelblichen Niederschlag, welcher mittels Filtration isoliert wurde, und einmal mit H₂O, einmal mit Ethanol, und einmal mit Aceton gewaschen und unter reduziertem Druck getrocknet wurde. Auf diese Art und Weise wurden 537 mg des Magnesiumsalzes der Formel 3k isoliert.

HPLC-Analyse: 92,4% Reinheit / 3,4 % N⁵-Methyl-THF.

UV (c = 20 mg/l in 0,1 M Phosphat-Puffer bei pH 7,3):

λₘₐₓ = 296 nm (ε = 27600) λₘᵢₙ = 244 nm Aₘₐₓ/Aₘᵢₙ = 3,68.

[α]_{D}= +12,1° (c = 1 in 0,1 N NaOH).

H₂O Gehalt (nach Karl Fischer): 8,9 %.

**Elementaranalyse * für CH₂-THF.Mg x 5 H₂O**

| | Berechneter Wert | exp. Wert |
|---|---|---|
| %C | 42,17 | 42,26 |
| %H | 5,49 | 5,51 |
| %N | 17,21 | 16,30 |
| %Mg | 4,27 | 5,02. |

*Probe getrocknet in der Luft: 15,8 % H₂O = Pentahydrat.

### Beispiel 10

Herstellung des tert.-Butylaminsalzes von (6RS)-N⁵,N¹⁰-Methylen-5,6,7,8-tetrahydrofolsäure der Formel 3f (Methode B).

1,0 g Kalziumsalz der Formel 3i wurde zu 15 ml einer 0,133 M Lösung des Oxalsäuresalzes von tert.-Butylamin hinzugegeben und bei einer Temperatur von +4°C unter Argon gerührt. Nach 30 Minuten Rühren bei gleicher Temperatur wurde der Kalziumoxalatniederschlag mittels Zentrifugation entfernt, und die erhaltene klare überstehende Lösung wurde langsam mit 75 ml Aceton unter Rühren bei einer Temperatur von +4°C verdünnt. Der erhaltene Niederschlag wurde mittels Filtration isoliert, einmal mit frischem Aceton gewaschen und unter reduziertem Druck getrocknet, um 965 mg der Verbindung der Formel 3f als ein gelbes Pulver zu ergeben.

HPLC-Analyse; 87,2 % Reinheit / 5,1 % N⁵-Methyl-THF.

[Weitere analytische Daten: siehe Beispiel 5].

### Beispiel 11

Herstellung des Magnesiumsalzes von (6RS)-N⁵,N¹⁰-Methylen-5,6,7,8-tetrahydrofolsäure der Formel 3k (Methode C).

65 g von 5,6,7,8-Tetrahydrofolsäure wurden in 400 ml Wasser suspendiert und der pH-Wert wurde auf einen Wert von 9,0 mittels der Hinzugabe von konzentriertem Ammoniak gebracht, wobei eine klare Lösung erhalten wurde. Dann wurden 13 ml einer 36%-igen wässrigen Formaldehydlösung hinzugetropft, und das Gemisch wurde während 10 Minuten bei Raumtemperatur stehengelassen.

Nach der Zugabe einer Lösung aus 50 g MgCl₂.6H₂O in 50 ml Wasser rührte man das so erhaltene Gemisch während 30 Minuten bei Raumtemperatur und liess es dann während 2 Stunden stehen. Dann wurde das gewünschte Produkt abgenutscht, und einmal mit Wasser und einmal mit Ethanol gewaschen. Nach dem Trocknen bei vermindertem Druck bei 50°C wurden 27 g des reinen N⁵,N¹⁰-Methylen-5,6,7,8-tetrahydrofolsäure-Magnesiumsalzes isoliert.

HPLC-Analyse: 98,8% Reinheit.

UV (c = 20 mg/l in 0,01 N NaOH):

λₘₐₓ = 296 nm (ε = 30200) λₘᵢₙ = 245 nm Aₘₐₓ/Aₘᵢₙ = 4,82.

[α]_{D}= +9,4° (c = 1 in 0,01 N NaOH).

### Beispiel 12

Herstellung des Calciumsalzes von (6RS)-N⁵,N¹⁰-Methylen-5,6,7,8-tetrahydrofolsäure der Formel 3i (Methode C).

190 g von 5,6,7,8-Tetrahydrofolsäure wurden in 1250 ml Wasser suspendiert. 90 ml konz. Ammoniak und 50 ml Pyridin wurden dann zugegeben, wobei man einen pH-Wert von 8,5 erreichte.

Nach der Zugabe von 40 ml wässriger, 36%-iger Formaldehydlösung wurde das Gemisch durch eine Schicht aus Aktivkohle und Celite filtriert.

Zur erhaltenen klaren Lösung wurden unter Rühren 200 ml einer 30%-igen Calciumchlorid Lösung zugegeben, und der pH-Wert wurde mittels der Zugabe von konz. Ammoniak auf einen Wert von 9,0 eingestellt.

Dann wurde auf eine Temperatur von +5°C abgekühlt, und man liess das Gemisch während 2 Stunden stehen. Anschliessend wurde abfiltriert, und das erhaltene Produkt wurde einmal mit Wasser und einmal mit Ethanol gewaschen. Nach dem Trocknen unter vermindertem Druck bei einer Temperatur von 50°C wurden 180 g des reinen N⁵,N¹⁰-Methylen-5,6,7,8-tetrahydrofolsäure-Calciumsalzes isoliert.

HPLC-Analyse: 96.5% Reinheit / 0,6 % N⁵-Methyl-THF.

UV (c = 20 mg/l in 0,01 N NaOH):

λₘₐₓ = 296 nm (ε = 26400) λₘᵢₙ = 245 nm Aₘₐₓ/Aₘᵢₙ = 4,87.

[α]_{D}= +17,3° (c = 1/ 0,01 N NaOH).

(6R)-und (6S)-N⁵,N¹⁰-Methylen-5,6,7,8-tetrahydrofolsäuresalze gemäss den Beispielen 2 - 11 wurden in derselben Art und Weise erhalten, wenn man jeweils von den (6R) und (6S) freien Säuren ausgeht.

### Stabilität der Verbindungen der Formeln 3c, 3d, 3e und 3f in 0,1 M Phosphat-Puffer

Eine 0,1 %ige Lösung in 0,1 M Phosphat-Puffer/pH 9,0 von jeder der Titelverbindungen wurde mittels HPLC bei den Zeiten 0, 60, 120, 300 Minuten analysiert, wobei jeweils 2,5 µl Injektionen getätigt wurden. Die prozentuale Wiedergewinnung bei der Zeit x (Rₓ) wurde erhalten mittels der folgenden Formel:

Rₓ = (B/A) . 100, wobei

A die integrierte CH₂ -THF Peak-Fläche zur Zeit 0 darstellt und

B die integrierte Peak-Fläche zur Zeit x darstellt.

| Zeit | Rₓ (3c) | Rₓ(3d) | Rₓ (3e) | Rₓ (3f) |
|---|---|---|---|---|
| 0 | 100 | 100 | 100 | 100 |
| 60 | 96,7 | 95,7 | 97,6 | 97,0 |
| 120 | 93,3 | 92,1 | 94,2 | 94,3 |
| 300 | 85,1 | - | 88,3 | 87,5 |

### Pharmazeutische Präparate für Injektionen

### a) Ampullen mit Flüssigkeiten

Die Verbindungen der Beispiele 2 bis 12, in einer Menge von 5-150 mg, wurden in einem pharmazeutisch annehmbaren Puffer, wie etwa 0,1 M Phosphat-Puffer, gelöst, so dass die Lösung einen pH-Wert von 9,0 hatte.

Die Lösung wurde bis zur Sterilität filtriert und in dunkle Glasampullen gefüllt, welche unter sterilen Bedingungen abgedichtet wurden.

### Beispiel

Eine Verbindung der Beispiele 2 - 12: 5 - 150 mg;

Pufferlösung, eingestellt auf pH 9,0 : 1 - 50 ml.

### b) Lyophilisierte Präparate

Die Verbindungen der Beispiele 2 bis 12, in einer Menge von 50-250 mg, wurden entweder in einem mazeutisch annehmbaren Puffer, wie etwa 0,1 M Phosphat-Puffer, oder in einer physiologischen Natriumchloridlösung gelöst. Die Lösung wurde bis zur Sterilität filtriert, in Proberöhrchen abgefüllt und in einer dem Fachmann bekannten Art lyophilisiert. Bei Bedarf kann das lyophilisierte Pulver wieder rekonstitutioniert werden mittels der Hinzugabe von sterilem Wasser vorgängig der Verabfolgung.

### Beispiel

Eine Verbindung der Beispiele 2 - 12: 50 - 250 mg;

entweder Pufferlösung oder physiologische Natriumchloridlösung: 3 - 20 ml.

Lyophilisieren der sterilen Lösung.

### Pharmazeutische Präparate für die orale Anwendung

Als ein Beispiel eines oralen Präparates werden Formulierungen für enterisch beschichtete Tabletten beschrieben, wobei klar ist, dass weitere orale Formen, wie etwa enterisch beschichtete Kapseln, in analoger Art und Weise hergestellt werden können.

### Enterisch beschichtete Tabletten

Die enterisch beschichteten Tabletten, welche von 5-30 mg einer der Komponenten der Beispiele 2-12 enthalten können, werden in der per se im Stand der Technik bekannten Art hergestellt, wobei die Enterobeschichtung ausgeführt wird gemäss "Remington Pharmaceutical Sciences", Seite 1614, 15th edition, 1975 und "Theory and Practice of Industrial Pharmacy", Lackman, Liberman, Canig, Seiten 116, 371, 470 - 2nd edition 1976.

### Beispiel

| | |
|---|---|
| Verbindung der Beispiele 1-12: | 5 bis 30 mg |
| Lactose USP: | 150 bis 125 mg |
| Stärke NF: | 35 bis 30 mg |
| Magnesiumstearat NF: | 3 mg |
| Eudragit NF: | 4 mg |
| Talc NF | 4 mg |
| Polyethylenglycol 6000 NF: | 0,4 mg. |

### Klinische Resultate und therapeutische Anwendungen von Salzen der N⁵,N¹⁰-Methylen-5,6,7,8-tetrahydrofolsäure

Als Beispiele der möglichen therapeutischen Anwendungen der beschriebenen Verbindungen liegen folgende klinische Ergebnisse vor:

### Patient A:

- Geschlecht:: weiblich
- Alter:: 19
- Diagnose:: osteogenes Sarkom mit Atmungs- und Schluckbeschwerden
- Behandlung:: 5-Fluorouracil intravenös verabreicht (300 - 750 mg/Woche) in Kombination mit intravenös verabreichtem N⁵,N¹⁰-Methylen-5,6,7,8-tetrahydrofolsäure-Calciumsalz (100 mg/Woche).

Klinischer Befund nach 5-wöchiger Behandlung:
- Sichtbare Reduktion des Tumorvolumens
- Verschwinden der Atmungs- und Schluckbeschwerden.

### Patient B:

- Geschlecht:: männlich
- Alter:: 52
- Diagnose:: Rektum Tumor mit Lebermetastasen
- Behandlung:: 5-Fluorouracil intravenös verabreicht (300 mg/Woche) in Kombination mit intravenös verabreichtem N⁵,N¹⁰-Methylen-5,6,7,8-tetrahydrofolsäure-Calciumsalz (100 mg/Woche).

Klinischer Befund nach 5-wöchiger Behandlung:
- Nekrose des Tumors und Ausscheidung des nekrotischen Gewebes
- Gewichtszunahme des Patienten.

Es sei festgestellt, dass eine vorherige Behandlung des Patienten mit equivalenten Mengen von 5-Fluorouracil und Leucovorin Calcium keine feststellbare Verbesserung des klinischen Zustands ergab.

### Patient C:

- Geschlecht:: weiblich
- Alter:: 53
- Diagnose:: Brustkarzinom links
- Behandlung:: 5-Fluorouracil intravenös verabreicht (2x 500 mg/Woche) während 3 Wochen und anschliessend (2 x 250 mg/Woche) während 2 Wochen in Kombination mit intravenös verabreichtem N⁵,N¹⁰-Methylen-5,6,7,8-tetrahydrofolsäure-Calciumsalz (100 mg/5-Fluorouracil Verabreichung).

Klinischer Befund nach 8 Wochen:
- Tumor praktisch nicht mehr sichtbar oder fassbar
- Heilende fibrotische Narben.

Die möglichen therapeutischen Anwendungen beschränken sich nicht auf die Behandlung der oben erwähnten Tumore mit Salzen der N⁵,N¹⁰-Methylen-5,6,7,8-tetrahydrofolsäure in Kombination mit 5-Fluorouracil, sondern schliessen auch Behandlungen von anderen Tumorarten mit Salzen der N⁵,N¹⁰-Methylen-5,6,7,8-tetrahydrofolsäure in Kombination mit 5-Fluorouracil oder anderen Chemotherapeutika, wie z.B. Floxuridin (FUDR), Methotrexat, Cisplatin, Adriamycin, Vincristin, u.s.w.,ein.

## Patentansprüche (Patentansprüche für folgende(n) Vertragsstaat(en): AT, BE, CH, DE, DK, FR, GB, IT, LI, LU, NL, SE)

1. Pharmazeutisch und/oder therapeutisch annehmbare Salze von N⁵,N¹⁰-Methylen-5,6,7,8-tetrahydrofolsäure der Formeln Ia und Ib 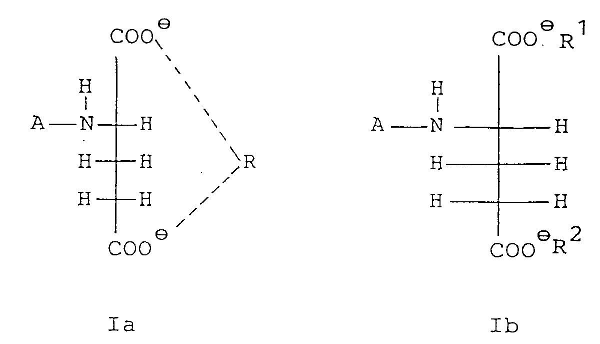 in Form des (6RS)-Diastereoisomerengemisches oder in Form der einzelnen (6R) oder (6S) Diastereoisomeren, worin
R ein mehrwertiges organisches Kation oder Ca⁺ oder Mg⁺ ist,
R¹ und R ein einwertiges organisches Kation sind, wobei R¹ und R gleich oder verschieden sein können, und
A einen Rest der Formel 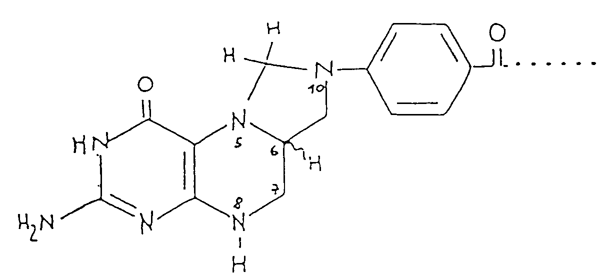
ist.

2. Salze nach Anspruch 1, dadurch gekennzeichnet, dass die organischen Kationen ausgewählt sind aus der Gruppe, bestehend aus Ethanolamin, Triethanolamin, 2-Dimethylaminoethanol, tert.-Butylamin, einer basischen Aminocarbonsäure, wie etwa Lysin, insbesondere (L)-Lysin, oder Arginin, insbesondere (L)-Arginin.

3. Verfahren zur Herstellung von Salzen nach einem der Ansprüche 1 bis 2, dadurch gekennzeichnet, dass man N⁵,N¹⁰-Methylen-5,6,7,8-tetrahydrofolsäure der Formel II 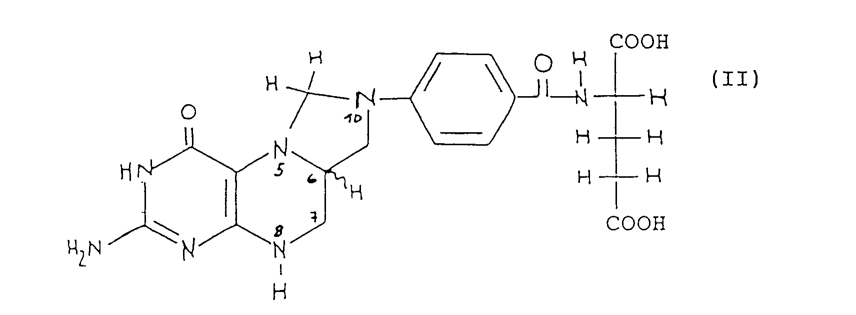 in Form des (6RS)-Diastereoisomerengemisches oder in Form der einzelnen (6R) oder (6S)Diastereoisomeren vorlegt und dann mit einer entsprechenden organischen und/oder anorganischen Base oder mit einem Ca⁺ oder Mg⁺ Salz zur Reaktion bringt.

4. Verfahren nach Anspruch 3, dadurch gekennzeichnet, dass die Reaktion in wenigstens einem Lösungmittel erfolgt, vorzugsweise ausgewählt aus Dimethylsulfoxid, Dimethylformamid, N,N-Dimethylacetamid, N-Methylpyrrolidon und Wasser.

5. Verfahren zur Herstellung von Salzen nach einem der Ansprüche 1 bis 2, dadurch gekennzeichnet, dass man ein Erdalkalimetallsalz der Formel III 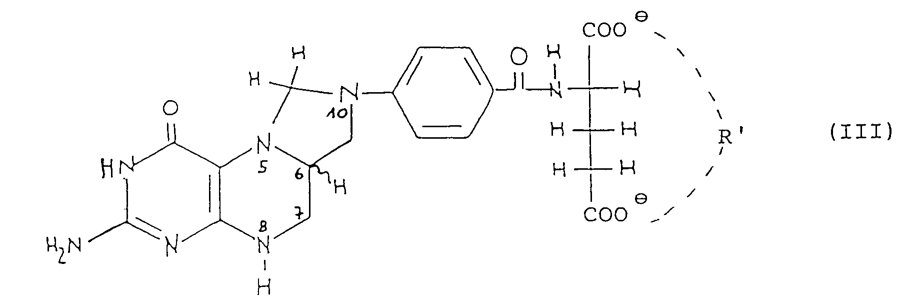 worin
R′ Ca⁺ oder Mg⁺ ist,
gemäss dem Verfahren nach einem der Ansprüche 3 bis 4 herstellt und dann mit einem entsprechenden Oxalsäuresalz zur Reaktion bringt, wobei das Erdalkalimetalloxalat ausfällt und wobei gleichzeitig das entsprechende organische und/oder anorganische Salz der Verbindung der Formel I gebildet wird, dann das ausgefällte Erdalkalimetalloxalat abtrennt, und dann aus der Lösung das gewünschte Salz der Formel I mittels Zugabe von wenigstens einem mit Wasser mischbaren organischen Lösungsmittel, beispielsweise Aceton, Methanol oder Ethanol, ausfällt.

6. Verfahren zur Herstellung von Erdalkalimetallsalzen von N⁵,N¹⁰-Methylen-5,6,7,8-tetrahydrofolsäure der Formel I' 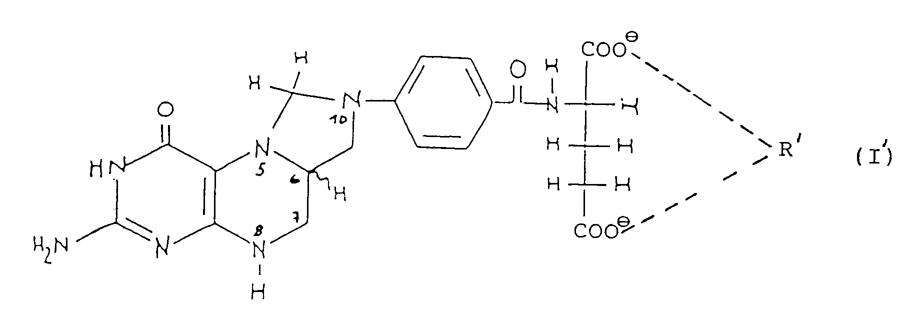 in Form des (6RS)-Diasteroisomerengemisches oder in Form der einzelnen (6R)-oder (6S)-Diastereoisomeren, worin
R′ Ca⁺ oder Mg⁺ ist,
dadurch gekennzeichnet, dass man eine wässrige Lösung eines wasserlöslichen Salzes der 5,6,7,8-Tetrahydrofolsäure der Formel IV 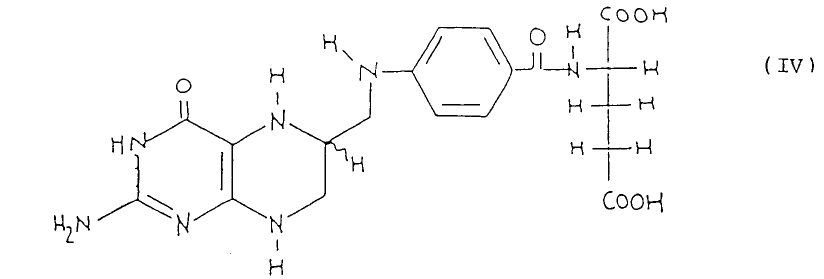 vorlegt und dann mit Formaldehyd zur Reaktion bringt, und anschliessend eine wässrige Lösung eines Ca⁺ oder Mg⁺ Salzes hinzugibt, wobei das entsprechende Erdalkalimetallsalz der Formel I' ausfällt.

7. Verfahren nach Anspruch 6, dadurch gekennzeichnet, dass man vor der Hinzugabe von Formaldehyd noch eine wasserlösliche, aromatische, Stickstoff enthaltende Base hinzugibt, welche insbesondere ausgewählt ist aus der Gruppe, bestehend aus Pyridin und substituierten Pyridinen, beispielsweise Picoline, Lutidine, Ethylpyridine, wobei Pyridin bevorzugt ist.

8. Verfahren nach einem der Ansprüche 6 bis 7, dadurch gekennzeichnet, dass man das wasserlösliche Salz der 5,6,7,8-Tetrahydrofolsäure herstellt durch Umsetzung der freien Säure in Wasser mit einer Verbindung, ausgewählt aus der Gruppe, bestehend aus Alkalihydroxiden, Alkalicarbonaten, Alkalihydrogencarbonaten, Ammoniak und organischen, Stickstoff enthaltenden Basen, wie beispielsweise Pyridin, Triethylamin, Triethanolamin.

9. Verfahren nach einem der Ansprüche 6 bis 8, dadurch gekennzeichnet, dass man nach der Hinzugabe der entsprechenden Erdalkalimetallsalzlösung den pH-Wert auf einen Wert im Bereich von 8,5 bis 9,5, insbesondere 9,0, einstellt, vorzugsweise mittels der Hinzugabe einer wässrigen Ammoniaklösung.

10. Verfahren nach einem der Ansprüche 6 bis 9, dadurch gekennzeichnet, dass man die Reaktion bei einer Temperatur im Bereich von 10°C bis 30°C, vorzugsweise bei Raumtemperatur, durchführt.

11. Verfahren nach einem der Ansprüche 6 bis 10, dadurch gekennzeichnet, dass man das Magnesiumsalz der N⁵,N¹⁰-Methylen-5,6,7,8-tetrahydrofolsäure herstellt durch Umsetzung bei Raumtemperatur der 5,6,7,8-Tetrahydrofolsäure in Wasser mit einer Verbindung, ausgewählt aus der Gruppe, bestehend aus Alkalihydroxiden, Alkalicarbonaten, Alkalihydrogencarbonaten, Ammoniak und organischen, Stickstoff enthaltenden Basen, wie beispielsweise Pyridin, Triethylamin, Triethanolamin,dann diese so hergestellte Salzlösung mit Formaldehyd zur Reaktion bringt, und anschliessend eine wässrige Lösung eines Magnesiumsalzes hinzugibt, wobei das Magnesiumsalz der N⁵,N¹⁰-Methylen-5,6,7,8-tetrahydrofolsäure ausfällt.

12. Verfahren nach einem der Ansprüche 6 bis 10, dadurch gekennzeichnet, dass man das Calciumsalz der N⁵,N¹⁰-Methylen-5,6,7,8-tetrahydrofolsäure herstellt durch Umsetzung bei Raumtemperatur der 5,6,7,8-Tetrahydrofolsäure in Wasser mit einer Verbindung, ausgewählt aus der Gruppe, bestehend aus Alkalihydroxiden, Alkalicarbonaten, Alkalihydrogencarbonaten, Ammoniak und organischen, Stickstoff enthaltenden Basen, wie beispielsweise Pyridin, Triethylamin, Triethanolamin,
dann zu dieser so hergestellten Salzlösung eine wasserlösliche, aromatische, Stickstoff enthaltende Base hinzugibt, welche insbesondere ausgewählt ist aus der Gruppe, bestehend aus Pyridin und substituierten Pyridinen, beispielsweise Picoline, Lutidine, Ethylpyridine, wobei Pyridin bevorzugt ist,
dann diese so hergestellte Salzlösung mit Formaldehyd zur Reaktion bringt, und anschliessend eine wässrige Lösung eines Calciumsalzes hinzugibt, wobei das Calciumsalz der N⁵,N¹⁰-Methylen-5,6,7,8-tetrahydrofolsäure ausfällt.

13. Arzneimittel für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen, dadurch gekennzeichnet, dass es als wenigstens eine aktive Verbindung wenigstens ein Salz nach einem der Ansprüche 1 bis 2 enthält, vorzugsweise in einer Menge von 1 mg bis 500 mg, insbesondere von 5 mg bis 150 mg, pro Dosiseinheit, und dass es vorzugsweise in der Form eines parenteralen und/oder oralen pharmazeutischen Präparates vorliegt.

14. Verwendung der Salze nach einem der Ansprüche 1 bis 2 zur Herstellung eines Arzneimittels für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen.

15. Verwendung nach Anspruch 14, dadurch gekennzeichnet, dass das Salz pro Dosiseinheit in einer Menge von 1 mg bis 500 mg, insbesondere von 5 mg bis 150 mg, im Arzneimittel vorhanden ist.

16. Verwendung nach einem der Ansprüche 14 bis 15, dadurch gekennzeichnet, dass das Arzneimittel in Form eines parenteralen und/oder oralen, pharmazeutischen Präparates vorliegt.

## Patentansprüche (Patentansprüche für folgende(n) Vertragsstaat(en): ES)

1. Verfahren zur Herstellung von pharmazeutisch und/oder therapeutisch annehmbaren Salzen von N⁵,N¹⁰-Methylen-5,6,7,8-tetrahydrofolsäure der Formeln Ia und Ib 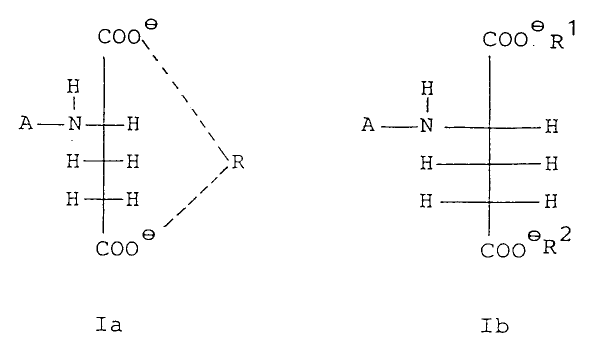 in Form des (6RS)-Diastereoisomerengemisches oder in Form der einzelnen (6R) oder (6S) Diastereoisomeren, worin
R ein mehrwertiges organisches Kation oder Ca⁺ oder Mg⁺ ist,
R¹ und R ein einwertiges organisches Kation sind, wobei R¹ und R gleich oder verschieden sein können, und
A einen Rest der Formel 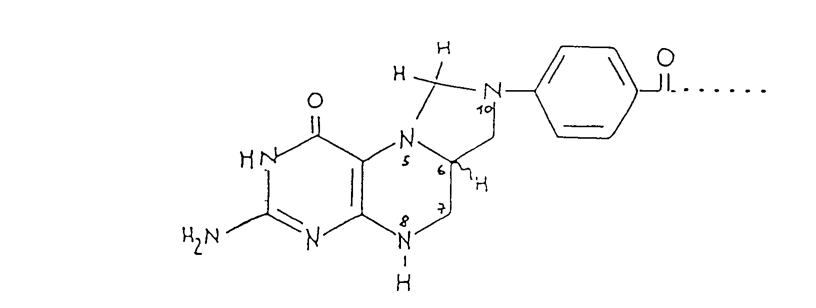
ist,
dadurch gekennzeichnet, dass man N⁵,N¹⁰-Methylen-5,6,7,8-tetrahydrofolsäure der Formel II 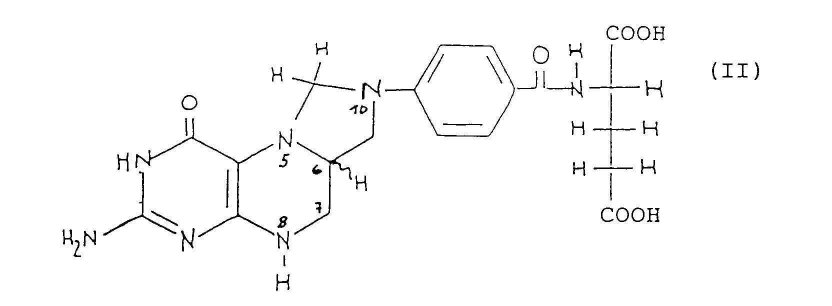 in Form des (6RS)-Diastereoisomerengemisches oder in Form der einzelnen (6R) oder (6S)-Diastereoisomeren vorlegt und dann mit einer entsprechenden organischen und/oder anorganischen Base oder mit einem Ca⁺ oder Mg⁺ Salz zur Reaktion bringt.

2. Verfahren nach Anspruch 1, dadurch gekennzeichnet, dass die Reaktion in wenigstens einem Lösungmittel erfolgt, vorzugsweise ausgewählt aus Dimethylsulfoxid, Dimethylformamid, N,N-Dimethylacetamid, N-Methylpyrrolidon und Wasser.

3. Verfahren nach einem der Ansprüche 1 bis 2, dadurch gekennzeichnet, dass die organischen Kationen ausgewählt sind aus der Gruppe, bestehend aus Ethanolamin, Triethanolamin, 2-Dimethylaminoethanol, tert.-Butylamin, einer basischen Aminocarbonsäure, wie etwa Lysin, insbesondere (L)-Lysin, oder Arginin, insbesondere (L)-Arginin.

4. Verfahren zur Herstellung von pharmazeutisch und/oder therapeutisch annehmbaren Salzen von N⁵,N¹⁰-Methylen-5,6,7,8-tetrahydrofolsäure der Formeln Ia und Ib 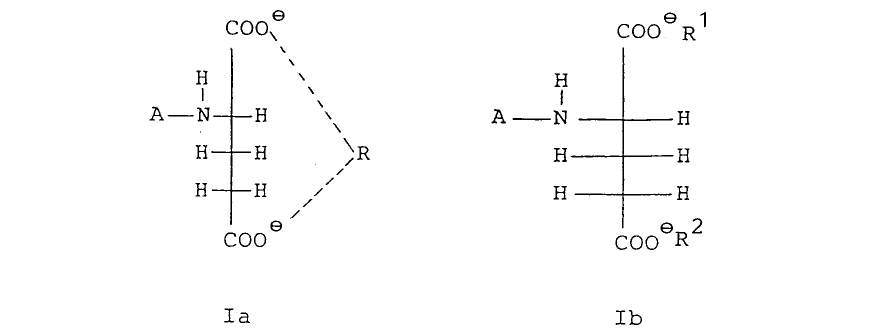 in Form des (6RS)-Diastereoisomerengemisches oder in Form der einzelnen (6R) oder (6S) Diastereoisomeren, worin
R ein mehrwertiges organisches Kation oder Ca⁺ oder Mg⁺ ist,
R¹ und R ein einwertiges organisches Kation sind, wobei R¹ und R gleich oder verschieden sein können, und
A einen Rest der Formel 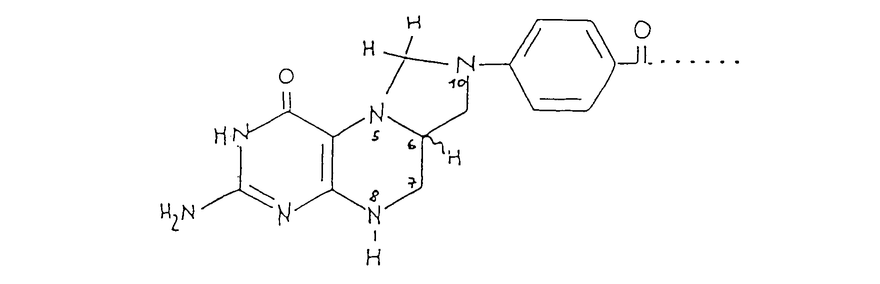
ist,
dadurch gekennzeichnet, dass man ein Erdalkalimetallsalz der Formel III 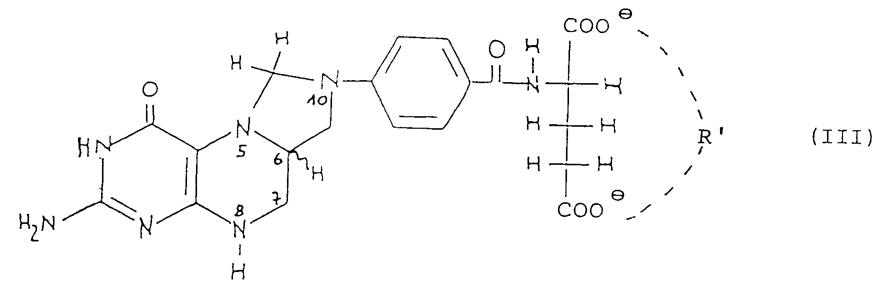 worin
R′ Ca⁺ oder Mg⁺ ist,
gemäss dem Verfahren nach einem der Ansprüche 1 bis 3 herstellt und dann mit einem entsprechenden Oxalsäuresalz zur Reaktion bringt, wobei das Erdalkalimetalloxalat ausfällt und wobei gleichzeitig das entsprechende organische und/oder anorganische Salz der Verbindung der Formel I gebildet wird, dann das ausgefällte Erdalkalimetalloxalat abtrennt, und dann aus der Lösung das gewünschte Salz der Formel I mittels Zugabe von wenigstens einem mit Wasser mischbaren organischen Lösungsmittel, beispielsweise Aceton, Methanol oder Ethanol, ausfällt.

5. Verfahren zur Herstellung von Erdalkalimetallsalzen von N⁵,N¹⁰-Methylen-5,6,7,8-tetrahydrofolsäure der Formel I' 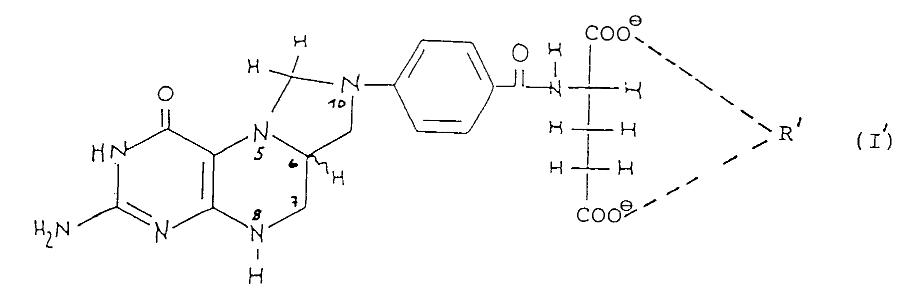 in Form des (6RS)-Diasteroisomerengemisches oder in Form der einzelnen (6R)-oder (6S)-Diastereoisomeren, worin
R′ Ca⁺ oder Mg⁺ ist,
dadurch gekennzeichnet, dass man eine wässrige Lösung eines wasserlöslichen Salzes der 5,6,7,8-Tetrahydrofolsäure der Formel IV 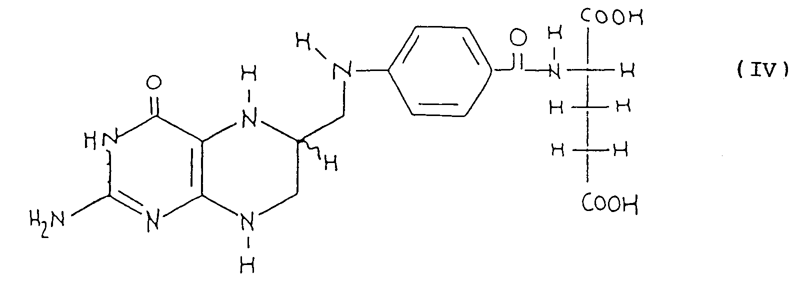 vorlegt und dann mit Formaldehyd zur Reaktion bringt, und anschliessend eine wässrige Lösung eines Ca⁺ oder Mg⁺ Salzes hinzugibt, wobei das entsprechende Erdalkalimetallsalz der Formel I' ausfällt.

6. Verfahren nach Anspruch 5, dadurch gekennzeichnet, dass man vor der Hinzugabe von Formaldehyd noch eine wasserlösliche, aromatische, Stickstoff enthaltende Base hinzugibt, welche insbesondere ausgewählt ist aus der Gruppe, bestehend aus Pyridin und substituierten Pyridinen, beispielsweise Picoline, Lutidine, Ethylpyridine, wobei Pyridin bevorzugt ist.

7. Verfahren nach einem der Ansprüche 5 bis 6, dadurch gekennzeichnet, dass man das wasserlösliche Salz der 5,6,7,8-Tetrahydrofolsäure herstellt durch Umsetzung der freien Säure in Wasser mit einer Verbindung, ausgewählt aus der Gruppe, bestehend aus Alkalihydroxiden, Alkalicarbonaten, Alkalihydrogencarbonaten, Ammoniak und organischen, Stickstoff enthaltenden Basen, wie beispielsweise Pyridin, Triethylamin, Triethanolamin.

8. Verfahren nach einem der Ansprüche 5 bis 7, dadurch gekennzeichnet, dass man nach der Hinzugabe der entsprechenden Erdalkalimetallsalzlösung den pH-Wert auf einen Wert im Bereich von 8,5 bis 9,5, insbesondere 9,0, einstellt, vorzugsweise mittels der Hinzugabe einer wässrigen Ammoniaklösung.

9. Verfahren nach einem der Ansprüche 5 bis 8, dadurch gekennzeichnet, dass man die Reaktion bei einer Temperatur im Bereich von 10°C bis 30°C, vorzugsweise bei Raumtemperatur, durchführt.

10. Verfahren nach einem der Ansprüche 5 bis 9, dadurch gekennzeichnet, dass man das Magnesiumsalz der N⁵,N¹⁰-Methylen-5,6,7,8-tetrahydrofolsäure herstellt durch Umsetzung bei Raumtemperatur der 5,6,7,8-Tetrahydrofolsäure in Wasser mit einer Verbindung, ausgewählt aus der Gruppe, bestehend aus Alkalihydroxiden, Alkalicarbonaten, Alkalihydrogencarbonaten, Ammoniak und organischen, Stickstoff enthaltenden Basen, wie beispielsweise Pyridin, Triethylamin, Triethanolamin, dann diese so hergestellte Salzlösung mit Formaldehyd zur Reaktion bringt, und anschliessend eine wässrige Lösung eines Magnesiumsalzes hinzugibt, wobei das Magnesiumsalz der N⁵,N¹⁰-Methylen-5,6,7,8-tetrahydrofolsäure ausfällt.

11. Verfahren nach einem der Ansprüche 5 bis 9, dadurch gekennzeichnet, dass man das Calciumsalz der N⁵,N¹⁰-Methylen-5,6,7,8-tetrahydrofolsäure herstellt durch Umsetzung bei Raumtemperatur der 5,6,7,8-Tetrahydrofolsäure in Wasser mit einer Verbindung, ausgewählt aus der Gruppe, bestehend aus Alkalihydroxiden, Alkalicarbonaten, Alkalihydrogencarbonaten, Ammoniak und organischen, Stickstoff enthaltenden Basen, wie beispielsweise Pyridin, Triethylamin, Triethanolamin,
dann zu dieser so hergestellten Salzlösung eine wasserlösliche, aromatische, Stickstoff enthaltende Base hinzugibt, welche insbesondere ausgewählt ist aus der Gruppe, bestehend aus Pyridin und substituierten Pyridinen, beispielsweise Picoline, Lutidine, Ethylpyridine, wobei Pyridin bevorzugt ist,
dann diese so hergestellte Salzlösung mit Formaldehyd zur Reaktion bringt, und anschliessend eine wässrige Lösung eines Calciumsalzes hinzugibt, wobei das Calciumsalz der N⁵,N¹⁰-Methylen-5,6,7,8-tetrahydrofolsäure ausfällt.

12. Verfahren zur Herstellung von Arzneimitteln für die synergistische Beeinflussung einer Krebs bekämpfenden Verbindung und/oder für die Verminderung der Toxizität einer Krebs bekämpfenden Verbindung und/oder zum Schutz menschlicher und/oder tierischen Zellen, dadurch gekennzeichnet, dass man im Arzneimittel, nebst üblichen Hilfs- und Trägerstoffen, als wenigstens eine aktive Verbindung, wenigstens ein Salz gemäss den vorhergehenden Ansprüchen einmischt, vorzugsweise in einer Menge von 1 mg bis 500 mg, insbesondere 5 mg bis 150 mg, pro Dosiseinheit, wobei das Arzneimittel in Form eines parenteralen und/oder oralen pharmazeutischen Präparates vorliegt, und als solches verabreicht wird.

## Patentansprüche (Patentansprüche für folgende(n) Vertragsstaat(en): GR)

1. Pharmazeutisch und/oder therapeutisch annehmbare Salze von N⁵,N¹⁰-Methylen-5,6,7,8-tetrahydrofolsäure der Formeln Ia und Ib 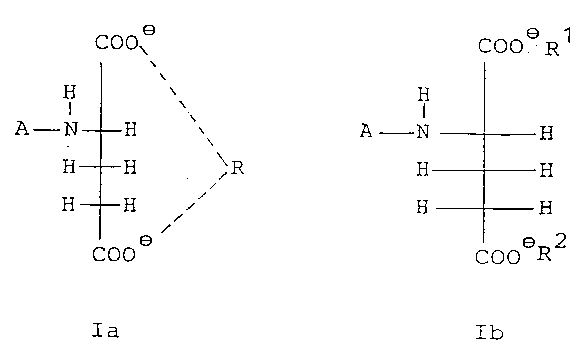 in Form des (6RS)-Diastereoisomerengemisches oder in Form der einzelnen (6R) oder (6S) Diastereoisomeren, worin
R ein mehrwertiges organisches Kation oder Ca⁺ oder Mg⁺ ist,
R¹ und R ein einwertiges organisches Kation sind, wobei R¹ und R gleich oder verschieden sein können, und
A einen Rest der Formel 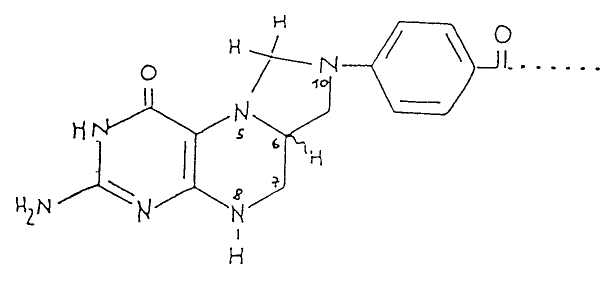
ist, die nach Anspruch 3 hergestellt werden.

2. Salze nach Anspruch 1, dadurch gekennzeichnet, dass die organischen Kationen ausgewählt sind aus der Gruppe, bestehend aus Ethanolamin, Triethanolamin, 2-Dimethylaminoethanol, tert.-Butylamin, einer basischen Aminocarbonsäure, wie etwa Lysin, insbesondere (L)-Lysin, oder Arginin, insbesondere (L)-Arginin.

3. Verfahren zur Herstellung von Salzen nach einem der Ansprüche 1 bis 2, dadurch gekennzeichnet, dass man N⁵,N¹⁰-Methylen-5,6,7,8-tetrahydrofolsäure der Formel II 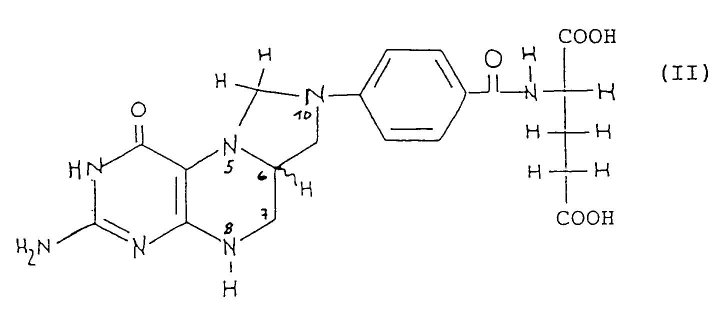 in Form des (6RS)-Diastereoisomerengemisches oder in Form der einzelnen (6R) oder (6S)-Diastereoisomeren vorlegt und dann mit einer entsprechenden organischen und/oder anorganischen Base oder mit einem Ca⁺ oder Mg⁺ Salz zur Reaktion bringt.

4. Verfahren nach Anspruch 3, dadurch gekennzeichnet, dass die Reaktion in wenigstens einem Lösungmittel erfolgt, vorzugsweise ausgewählt aus Dimethylsulfoxid, Dimethylformamid, N,N-Dimethylacetamid, N-Methylpyrrolidon und Wasser.

5. Verfahren zur Herstellung von Salzen nach einem der Ansprüche 1 bis 2, dadurch gekennzeichnet, dass man ein Erdalkalimetallsalz der Formel III 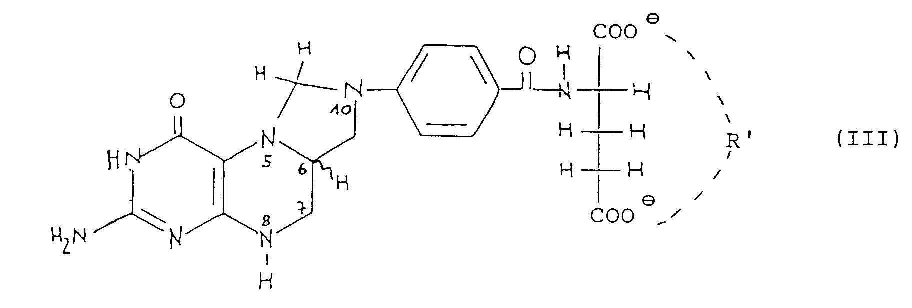 worin
R′ Ca⁺ oder Mg⁺ ist,
gemäss dem Verfahren nach einem der Ansprüche 3 bis 4 herstellt und dann mit einem entsprechenden Oxalsäuresalz zur Reaktion bringt, wobei das Erdalkalimetalloxalat ausfällt und wobei gleichzeitig das entsprechende organische und/oder anorganische Salz der Verbindung der Formel I gebildet wird, dann das ausgefällte Erdalkalimetalloxalat abtrennt, und dann aus der Lösung das gewünschte Salz der Formel I mittels Zugabe von wenigstens einem mit Wasser mischbaren organischen Lösungsmittel, beispielsweise Aceton, Methanol oder Ethanol, ausfällt.

6. Verfahren zur Herstellung von Erdalkalimetallsalzen von N⁵,N¹⁰-Methylen-5,6,7,8-tetrahydrofolsäure der Formel I' 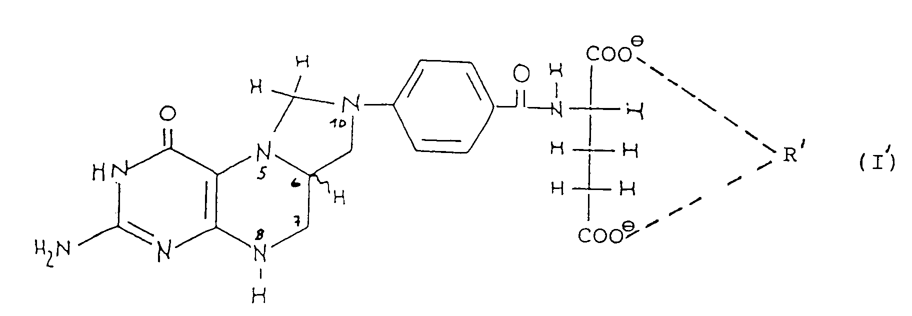 in Form des (6RS)-Diasteroisomerengemisches oder in Form der einzelnen (6R)-oder (6S)-Diastereoisomeren, worin
R′ Ca⁺ oder Mg⁺ ist,
dadurch gekennzeichnet, dass man eine wässrige Lösung eines wasserlöslichen Salzes der 5,6,7,8-Tetrahydrofolsäure der Formel IV 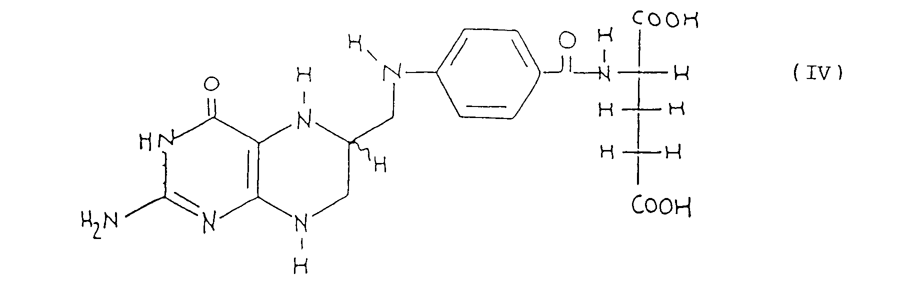 vorlegt und dann mit Formaldehyd zur Reaktion bringt, und anschliessend eine wässrige Lösung eines Ca⁺ oder Mg⁺ Salzes hinzugibt, wobei das entsprechende Erdalkalimetallsalz der Formel I' ausfällt.

7. Verfahren nach Anspruch 6, dadurch gekennzeichnet, dass man vor der Hinzugabe von Formaldehyd noch eine wasserlösliche, aromatische, Stickstoff enthaltende Base hinzugibt, welche insbesondere ausgewählt ist aus der Gruppe, bestehend aus Pyridin und substituierten Pyridinen, beispielsweise Picoline, Lutidine, Ethylpyridine, wobei Pyridin bevorzugt ist.

8. Verfahren nach einem der Ansprüche 6 bis 7, dadurch gekennzeichnet, dass man das wasserlösliche Salz der 5,6,7,8-Tetrahydrofolsäure herstellt durch Umsetzung der freien Säure in Wasser mit einer Verbindung, ausgewählt aus der Gruppe, bestehend aus Alkalihydroxiden, Alkalicarbonaten, Alkalihydrogencarbonaten, Ammoniak und organischen, Stickstoff enthaltenden Basen, wie beispielsweise Pyridin, Triethylamin, Triethanolamin.

9. Verfahren nach einem der Ansprüche 6 bis 8, dadurch gekennzeichnet, dass man nach der Hinzugabe der entsprechenden Erdalkalimetallsalzlösung den pH-Wert auf einen Wert im Bereich von 8,5 bis 9,5, insbesondere 9,0, einstellt, vorzugsweise mittels der Hinzugabe einer wässrigen Ammoniaklösung.

10. Verfahren nach einem der Ansprüche 6 bis 9, dadurch gekennzeichnet, dass man die Reaktion bei einer Temperatur im Bereich von 10°C bis 30°C, vorzugsweise bei Raumtemperatur, durchführt.

11. Verfahren nach einem der Ansprüche 6 bis 10, dadurch gekennzeichnet, dass man das Magnesiumsalz der N⁵,N¹⁰-Methylen-5,6,7,8-tetrahydrofolsäure herstellt durch Umsetzung bei Raumtemperatur der 5,6,7,8-Tetrahydrofolsäure in Wasser mit einer Verbindung, ausgewählt aus der Gruppe, bestehend aus Alkalihydroxiden, Alkalicarbonaten, Alkalihydrogencarbonaten, Ammoniak und organischen, Stickstoff enthaltenden Basen, wie beispielsweise Pyridin, Triethylamin, Triethanolamin, dann diese so hergestellte Salzlösung mit Formaldehyd zur Reaktion bringt, und anschliessend eine wässrige Lösung eines Magnesiumsalzes hinzugibt, wobei das Magnesiumsalz der N⁵,N¹⁰-Methylen-5,6,7,8-tetrahydrofolsäure ausfällt.

12. Verfahren nach einem der Ansprüche 6 bis 10, dadurch gekennzeichnet, dass man das Calciumsalz der N⁵,N¹⁰-Methylen-5,6,7,8-tetrahydrofolsäure herstellt durch Umsetzung bei Raumtemperatur der 5,6,7,8-Tetrahydrofolsäure in Wasser mit einer Verbindung, ausgewählt aus der Gruppe, bestehend aus Alkalihydroxiden, Alkalicarbonaten, Alkalihydrogencarbonaten, Ammoniak und organischen, Stickstoff enthaltenden Basen, wie beispielsweise Pyridin, Triethylamin, Triethanolamin,
dann zu dieser so hergestellten Salzlösung eine wasserlösliche, aromatische, Stickstoff enthaltende Base hinzugibt, welche insbesondere ausgewählt ist aus der Gruppe, bestehend aus Pyridin und substituierten Pyridinen, beispielsweise Picoline, Lutidine, Ethylpyridine, wobei Pyridin bevorzugt ist,
dann diese so hergestellte Salzlösung mit Formaldehyd zur Reaktion bringt, und anschliessend eine wässrige Lösung eines Calciumsalzes hinzugibt, wobei das Calciumsalz der N⁵,N¹⁰-Methylen-5,6,7,8-tetrahydrofolsäure ausfällt.

13. Verwendung der Salze, die nach Anspruch 3 hergestellt werden, zur Herstellung eines Arzneimittels für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen.

14. Verwendung nach Anspruch 13, dadurch gekennzeichnet, dass das Salz pro Dosiseinheit in einer Menge von 1 mg bis 500 mg, insbesondere von 5 mg bis 150 mg, im Arzneimittel vorhanden ist.

15. Verwendung nach einem der Ansprüche 13 bis 14, dadurch gekennzeichnet, dass das Arzneimittel in Form eines parenteralen und/oder oralen, pharmazeutischen Präparates vorliegt.

## Claims (Claims for the following Contracting State(s): AT, BE, CH, DE, DK, FR, GB, IT, LI, LU, NL, SE)

1. Pharmaceutical and/or therapeutical acceptable salts of N⁵,N¹⁰-methylene-5,6,7,8-tetrahydrofolic acid of the formulae Ia and Ib 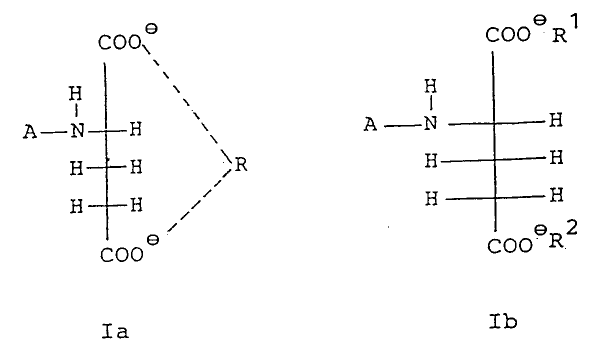 in the form of the mixture of (6RS)-diastereoisomers or in the form of the single (6R) or (6S) diastereoisomers, wherein
R is a multivalent organic cation or Ca⁺ or Mg⁺,
R¹ and R are a monovalent organic cation, whereby R¹ and R may be the same or be different, and
A is a residue of the formula

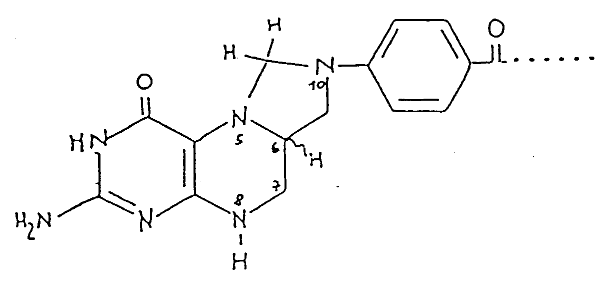

2. Salts according to claim 1, characterized in that the organic cations are selected from the group consisting of ethanolamine, triethanolamine, 2-dimethylaminoethanol, tert.-butylamine, a basic amino carboxylic acid, such as lysine, especially (L)-lysine, or arginine, especially (L)-arginine.

3. A process for the preparation of salts according to one of claims 1 to 2, characterized in that N⁵,N¹⁰-methylene-5,6,7,8-tetrahydrofolic acid of the formula II 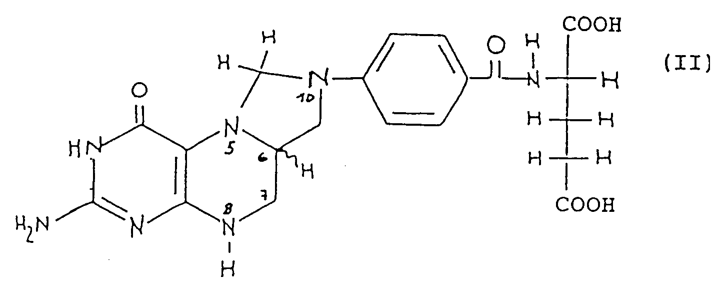 in the form of the mixture of the (6RS)-diastereoisomers or in the form of the single (6R) or (6S)-diastereoisomers is submitted, and then reacted with a corresponding organic and/or inorganic base or with a Ca⁺ or Mg⁺ salt.

4. The process according to claim 3, characterized in that the reaction is carried out in at least one solvent, preferably selected from dimethylsulfoxide, dimethylformamide, N,N-dimethylacetamide, N-methylpyrrolidon and water.

5. A process for the preparation of salts according to one of claims 1 to 2, characterized in that an alkaline earth metal salt of the formula III 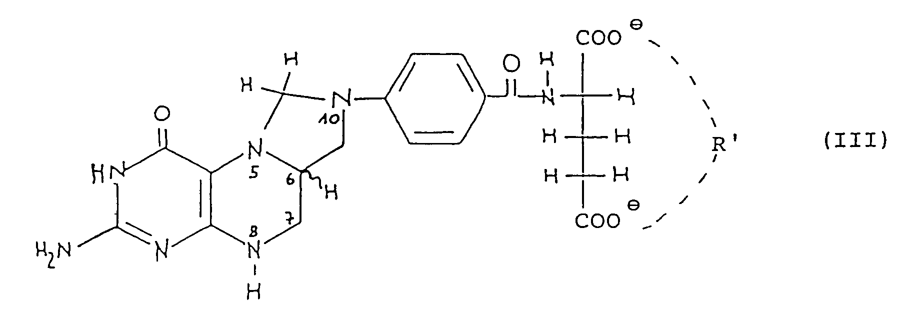 wherein
R' is Ca⁺ or Mg⁺,
is prepared according to the process according to one of claims 3 to 4, and then is reacted with a corresponding oxalic acid salt, whereby the alkaline earth metal oxalate precipitates, and whereby simultaneously the corresponding organic and/or inorganic salt of the compound of the formula I is formed, then separating the precipitated alkaline earth metal oxalate, and then precipitating from the solution the desired salt of formula I by means of the addition of at least one with water mixable organic solvent, for example acetone, methanol or ethanol.

6. A process for the preparation of alkaline earth metal salts of N⁵,N¹⁰-methylene-5,6,7,8-tetrahydrofolic acid of the formula I' 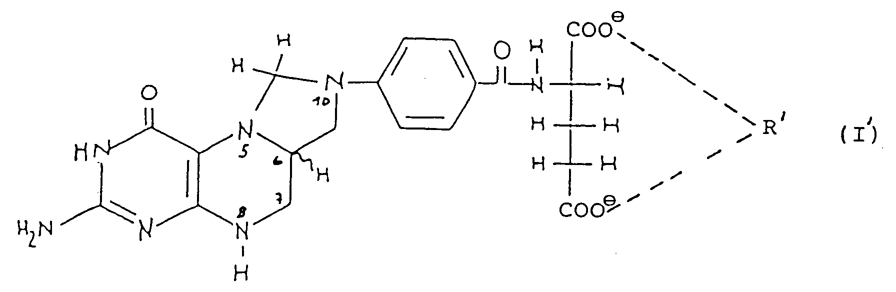 in the form of the mixture of the (6RS)-diastereoisomers or in the form of the single (6R) or (6S)-diastereoisomers,
wherein
R' is Ca⁺ or Mg⁺,
characterized in that an aqueous solution of a water soluble salt of the 5,6,7,8-tetrahydrofolic acid of the formula IV 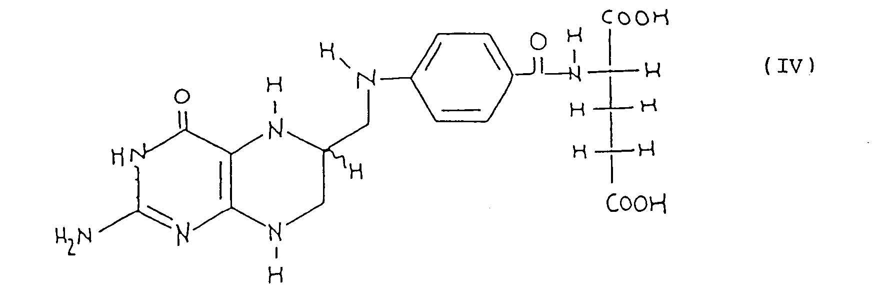 is submitted and then reacted with formaldehyde, and then is added an aqueous solution of a Ca⁺ or Mg⁺ salt, whereby the corresponding alkaline earth metal salt of the formula I' precipitates.

7. The process according to claim 6, characterized in that prior to the addition of formaldehyde a watersoluble, aromatic, nitrogen containing base is added, which is especially selected from the group consisting of pyridine and substituted pyridines, for example picolines, lutidines, ethylpyridines, whereby pyridine is preferred.

8. The process according to one of claims 6 to 7, characterized in that the watersoluble salt of the 5,6,7,8-tetrahydrofolic acid is prepared by reacting the free acid in water together with a compound selected from the group, consisting of alkalihydroxides, alkalicarbonates, alkalihydrogencarbonates, ammonia and organic, nitrogen containing bases, such as pyridine, triethylamine, triethanolamine.

9. The process according to one of claims 6 to 8, characterized in that after the addition of the corresponding alkaline earth metal salt solution the pH-value is adjusted to a value in the range from 8.5 to 9.5, especially 9.0, preferably by means of the addition of an aqueous ammonia solution.

10. The process according to one of claims 6 to 9, characterized in that the reaction is carried out at a temperature in the range from 10°C to 30°C, preferably at room temperature.

11. The process according to one of claims 6 to 10, characterized in that the magnesium salt of the N⁵,N¹⁰-methylene-5,6,7,8-tetrahydrofolic acid is prepared by reacting at room temperature the 5,6,7,8-tetrahydrofolic acid in water with a compound, selected from the group, consisting of alkalihydroxides, alkalicarbonates, alkalihydrogencarbonates, ammonia and organic, nitrogen containing bases, such as for example pyridine, triethylamine, triethanolamine, then reacting the so prepared salt solution with formaldehyde, and then adding an aqueous solution of a magnesium salt, whereby the magnesium salt of the N⁵,N¹⁰-methylene-5,6,7,8-tetrahydrofolic acid precipitates.

12. The process according to one of claims 6 to 10, characterized in that the calcium salt of the N⁵,N¹⁰-methylene-5,6,7,8-tetrahydrofolic acid is prepared by reacting at room temperature the 5,6,7,8-tetrahydrofolic acid in water with a compound, selected from the group, consisting of alkalihydroxides, alkalicarbonates, alkalihydrogencarbonates, ammonia and organic, nitrogen containing bases, such as for example pyridine, triethylamine, triethanolamine,
then adding to the so prepared salt solution a watersoluble, aromatic, nitrogen containing base, which is especially selected from the group, consisting of pyridine and substituted pyridines, for example picolines, lutidines, ethylpyridines, whereby pyridine is preferred,
then reacting the so prepared salt solution with formaldehyde, and then adding an aqueous solution of a calcium salt, whereby the calcium salt of the N⁵,N¹⁰-methylene-5,6,7,8-tetrahydrofolic acid precipitates.

13. A medicament for the synergistic excertion of influence of a cancer controlling compound and/or for the reduction of the toxicity of a cancer controlling compound and/or for the protection of human and/or animal cells, characterized in that it contains as at least one active compound at least one salt according to one of claims 1 to 2, preferably in an amount from 1 mg to 500 mg, especially from 5 mg to 150 mg, per dosis unit, and that it is preferably in the form of a parenteral and/or oral pharmaceutical preparation.

14. Use of the salts according to one of claims 1 to 2 for the preparation of a medicament for the synergistic excertion of influence of a cancer controlling compound and/or for the reduction of the toxicity of a cancer controlling compound and/or for the protection of human and/or animal cells.

15. Use according to claim 14, characterized in that the salt is present per dosis unit in the medicament in an amount from 1 mg to 500 mg, especially from 5 mg to 150 mg.

16. Use according to one of claims 14 to 15, characterized in that the medicament is in the form of a parenteral and/or oral, pharmaceutical preparation.

## Claims (Claims for the following Contracting State(s): ES)

1. A process for the preparation of pharmaceutical and/or therapeutical acceptable salts of N⁵,N¹⁰-methylene-5,6,7,8-tetrahydrofolic acid of the formulae Ia and Ib 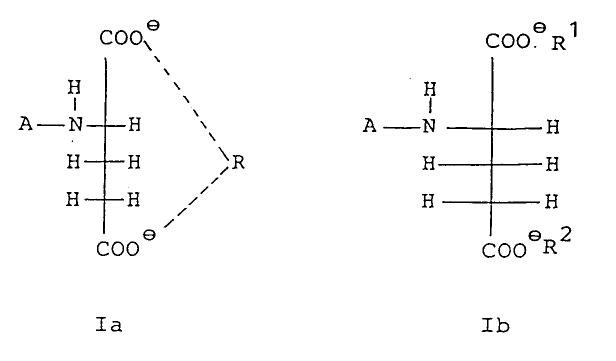 in the form of the mixture of the (6RS)-diastereoisomers or in the form of the single (6R) or (6S) diastereoisomers, wherein
R is a multivalent organic cation or Ca⁺ or Mg⁺,
R¹ and R are a monovalent organic cation, whereby R¹ and R may be the same or be different, and
A is a residue of the formula 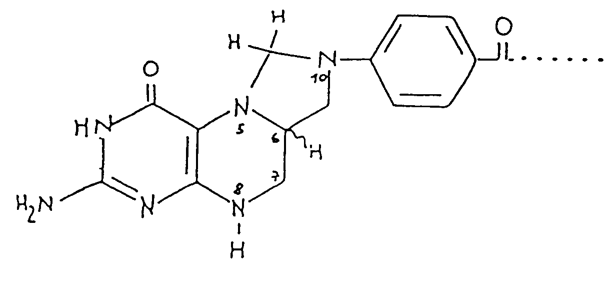
characterized in that N⁵,N¹⁰-methylene-5,6,7,8-tetrahydrofolic acid of the formula II 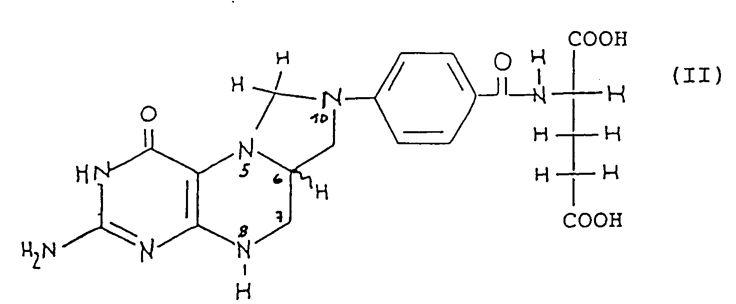 in the form of the mixture of the (6RS)-diastereoisomers or in the form of the single (6R) or (6S)-diastereoisomers is submitted, and then reacted with a corresponding organic and/or inorganic base or with a Ca⁺ or Mg⁺ salt.

2. The process according to claim 1, characterized in that the reaction is carried out in at least one solvent, preferably selected from dimethylsulfoxide, dimethylformamide, N,N-dimethylacetamide, N-methylpyrrolidon and water.

3. The process according to one of claims 1 to 2, characterized in that the organic cations are selected from the group consisting of ethanolamine, triethanolamine, 2-dimethylaminoethanol, tert.-butylamine, a basic amino carboxylic acid, such as lysine, especially (L)-lysine, or arginine, especially (L)-arginine.

4. A process for the preparation of pharmaceutical and/or therapeutical acceptable salts of N⁵,N¹⁰-methylene-5,6,7,8-tetrahydrofolic acid of the formulae Ia and Ib 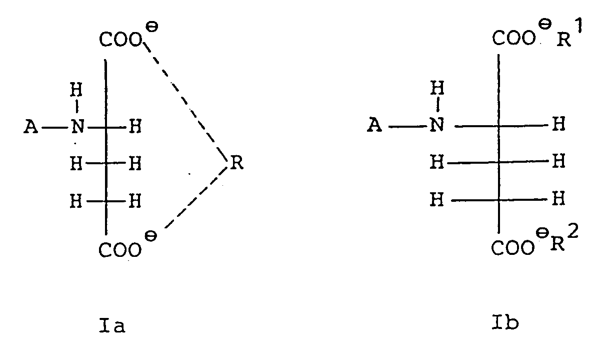 in the form of the mixture of the (6RS)-diastereoisomers or in the form of the single (6R) or (6S) diastereoisomers, wherein
R is a multivalent organic cation or Ca⁺ or Mg⁺,
R¹ and R are a monovalent organic cation, whereby R¹ and R may be the same or be different, and
A is a residue of the formula 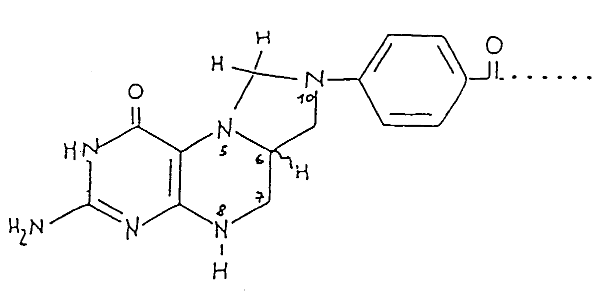
characterized in that an alkaline earth metal salt of the formula III 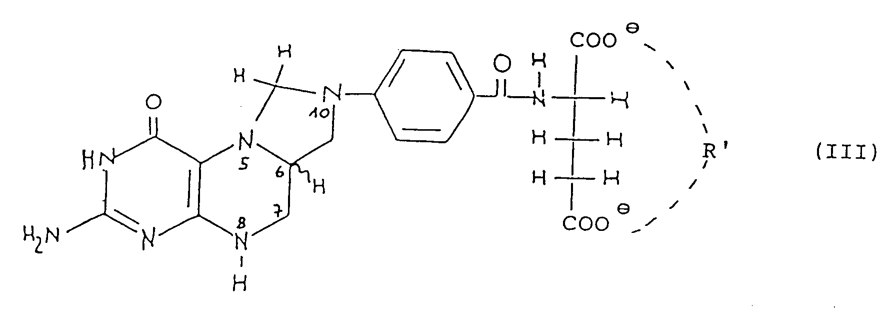 wherein
R′ is Ca⁺ or Mg⁺,
is prepared according to the process according to one of claims 1 to 3, and then is reacted with a corresponding oxalic acid salt, whereby the alkaline earth metal oxalate precipitates, and whereby simultaneously the corresponding organic and/or inorganic salt of the compound of the formula I is formed, then separating the precipitated alkaline earth metal oxalate, and then precipitating from the solution the desired salt of formula I by means of the addition of at least one with water mixable organic solvent, for example acetone, methanol or ethanol.

5. A process for the preparation of alkaline earth metal salts of N⁵,N¹⁰-methylene-5,6,7,8-tetrahydrofolic acid of the formula I' 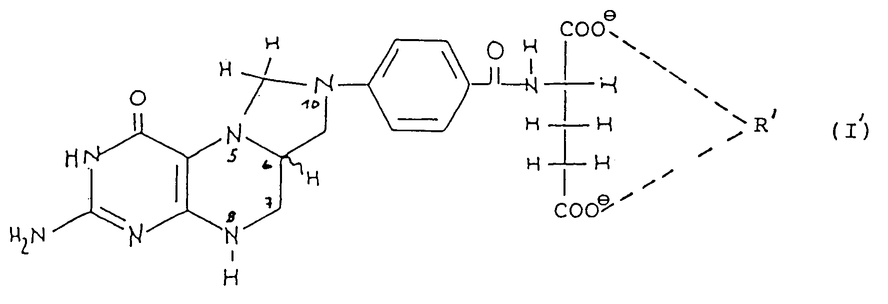 in the form of the mixture of the (6RS)-diastereoisomers or in the form of the single (6R) or (6S)-diastereoisomers,
wherein
R' is Ca⁺ or Mg⁺,
characterized in that an aqueous solution of a water soluble salt of the 5,6,7,8-tetrahydrofolic acid of the formula IV 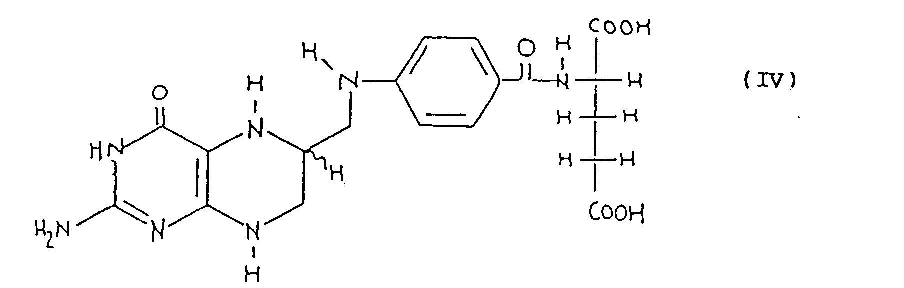 is submitted and then reacted with formaldehyde, and then is added an aqueous solution of a Ca⁺ or Mg⁺ salt, whereby the corresponding alkaline earth metal salt of the formula I' precipitates.

6. The process according to claim 5, characterized in that prior to the addition of formaldehyde a watersoluble, aromatic, nitrogen containing base is added, which is especially selected from the group consisting of pyridine and substituted pyridines, for example picolines, lutidines, ethylpyridines, whereby pyridine is preferred.

7. The process according to one of claims 5 to 6, characterized in that the watersoluble salt of the 5,6,7,8-tetrahydrofolic acid is prepared by reacting the free acid in water together with a compound selected from the group, consisting of alkalihydroxides, alkalicarbonates, alkalihydrogencarbonates, ammonia and organic, nitrogen containing bases, such as pyridine, triethylamine, triethanolamine.

8. The process according to one of claims 5 to 7, characterized in that after the addition of the corresponding alkaline earth metal salt solution the pH-value is adjusted to a value in the range from 8.5 to 9.5, especially 9.0, preferably by means of the addition of an aqueous ammonia solution.

9. The process according to one of claims 5 to 8, characterized in that the reaction is carried out at a temperature in the range from 10°C to 30°C, preferably at room temperature.

10. The process according to one of claims 5 to 9, characterized in that the magnesium salt of the N⁵,N¹⁰-methylene-5,6,7,8-tetrahydrofolic acid is prepared by reacting at room temperature the 5,6,7,8-tetrahydrofolic acid in water with a compound, selected from the group, consisting of alkalihydroxides, alkalicarbonates, alkalihydrogencarbonates, ammonia and organic, nitrogen containing bases, such as for example pyridine, triethylamine, triethanolamine, then reacting the so prepared salt solution with formaldehyde, and then adding an aqueous solution of a magnesium salt, whereby the magnesium salt of the N⁵,N¹⁰-methylene-5,6,7,8-tetrahydrofolic acid precipitates.

11. The process according to one of claims 5 to 9, characterized in that the calcium salt of the N⁵,N¹⁰-methylene-5,6,7,8-tetrahydrofolic acid is prepared by reacting at room temperature the 5,6,7,8-tetrahydrofolic acid in water with a compound, selected from the group, consisting of alkalihydroxides, alkalicarbonates, alkalihydrogencarbonates, ammonia and organic, nitrogen containing bases, such as for example pyridine, triethylamine, triethanolamine,
then adding to the so prepared salt solution a watersoluble, aromatic, nitrogen containing base, which is especially selected from the group, consisting of pyridine and substituted pyridines, for example picolines, lutidines, ethylpyridines, whereby pyridine is preferred,
then reacting the so prepared salt solution with formaldehyde, and then adding an aqueous solution of a calcium salt, whereby the calcium salt of the N⁵,N¹⁰-methylene-5,6,7,8-tetrahydrofolic acid precipitates.

12. A process for the preparation of medicaments for the synergistic excertion of influence of a cancer controlling compound and/or for the reduction of the toxicity of a cancer controlling compound and/or for the protection of human and/or animal cells, characterized in that the medicament is admixed, besides usual adjuvants and carrier materials, as at least one active compound, with at least one salt according to the preceding claims, preferably in an amount from 1 mg to 500 mg, especially from 5 mg to 150 mg, per dosis unit, whereby the medicament is in the form of a parenteral and/or oral pharmaceutical preparation, and is administered as such.

## Claims (Claims for the following Contracting State(s): GR)

1. Pharmaceutical and/or therapeutical acceptable salts of N⁵,N¹⁰-methylene-5,6,7,8-tetrahydrofolic acid of the formulae Ia and Ib 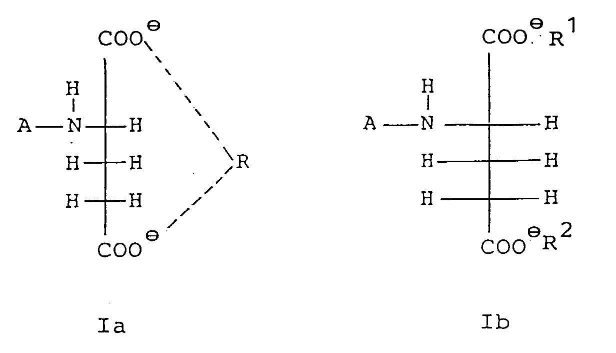 in the form of the mixture of the (6RS)-diastereoisomers or in the form of the single (6R) or (6S) diastereoisomers, wherein
R is a multivalent organic cation or Ca⁺ or Mg⁺,
R¹ and R are a monovalent organic cation, whereby R¹ and R may be the same or be different, and
A is a residue of the formula 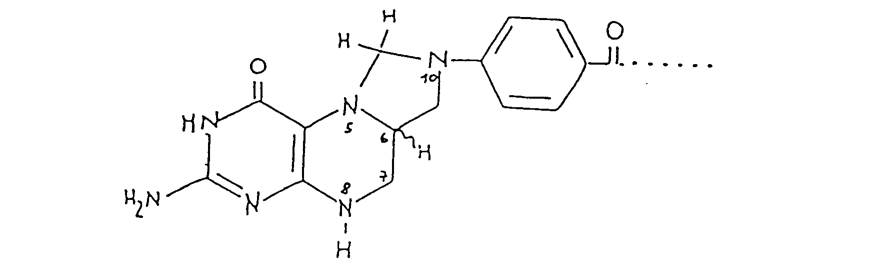
which are prepared according to claim 3.

2. Salts according to claim 1, characterized in that the organic cations are selected from the group consisting of ethanolamine, triethanolamine, 2-dimethylaminoethanol, tert.-butylamine, a basic amino carboxylic acid, such as lysine, especially (L)-lysine, or arginine, especially (L)-arginine.

3. A process for the preparation of salts according to one of claims 1 to 2, characterized in that N⁵,N¹⁰-methylene-5,6,7,8-tetrahydrofolic acid of the formula II 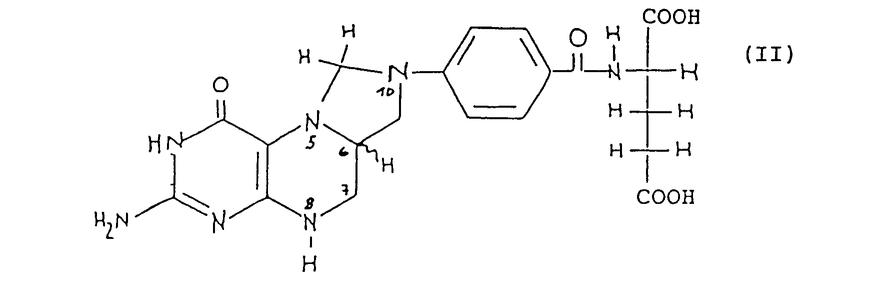 in the form of the mixture of the (6RS)-diastereoisomers or in the form of the single (6R) or (6S)-diastereoisomers is submitted, and then reacted with a corresponding organic and/or inorganic base or with a Ca⁺ or Mg⁺ salt.

4. The process according to claim 3, characterized in that the reaction is carried out in at least one solvent, preferably selected from dimethylsulfoxide, dimethylformamide, N,N-dimethylacetamide, N-methylpyrrolidon and water.

5. A process for the preparation of salts according to one of claims 1 to 2, characterized in that an alkaline earth metal salt of the formula III 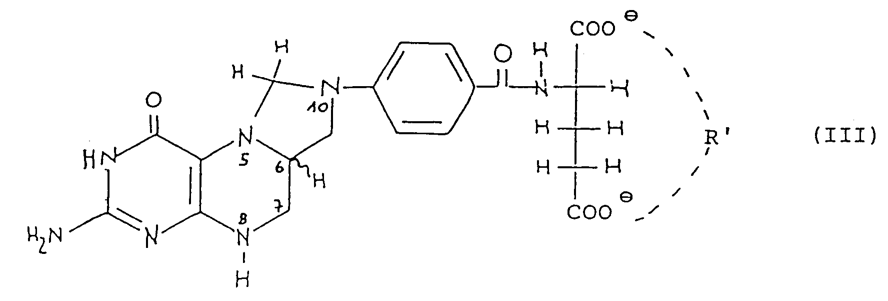 wherein
R′ is Ca⁺ or Mg⁺,
is prepared according to the process according to one of claims 3 to 4, and then is reacted with a corresponding oxalic acid salt, whereby the alkaline earth metal oxalate precipitates, and whereby simultaneously the corresponding organic and/or inorganic salt of the compound of the formula I is formed, then separating the precipitated alkaline earth metal oxalate, and then precipitating from the solution the desired salt of formula I by means of the addition of at least one with water mixable organic solvent, for example acetone, methanol or ethanol.

6. A process for the preparation of alkaline earth metal salts of N⁵,N¹⁰-methylene-5,6,7,8-tetrahydrofolic acid of the formula I' 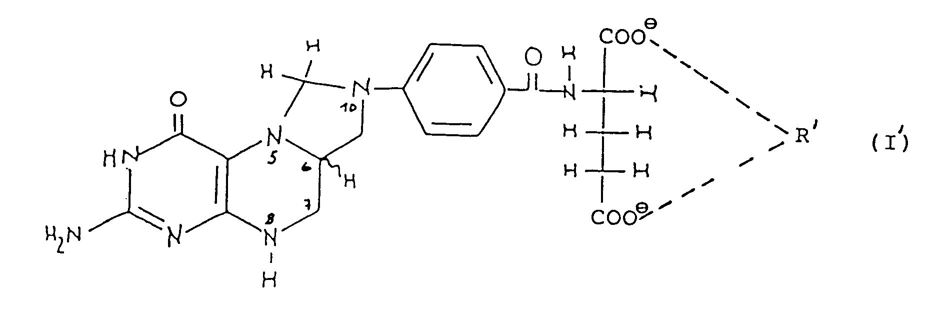 in the form of the mixture of the (6RS)-diastereoisomers or in the form of the single (6R) or (6S)-diastereoisomers, wherein
R′ is Ca⁺ or Mg⁺,
characterized in that an aqueous solution of a water soluble salt of the 5,6,7,8-tetrahydrofolic acid of the formula IV 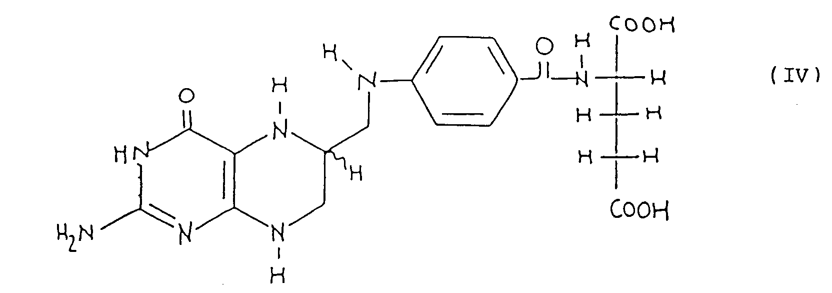 is submitted and then reacted with formaldehyde, and then is added an aqueous solution of a Ca⁺ or Mg⁺ salt, whereby the corresponding alkaline earth metal salt of the formula I' precipitates.

7. The process according to claim 6, characterized in that prior to the addition of formaldehyde a watersoluble, aromatic, nitrogen containing base is added, which is especially selected from the group consisting of pyridine and substituted pyridines, for example picolines, lutidines, ethylpyridines, whereby pyridine is preferred.

8. The process according to one of claims 6 to 7, characterized in that the watersoluble salt of the 5,6,7,8-tetrahydrofolic acid is prepared by reacting the free acid in water together with a compound selected from the group, consisting of alkalihydroxides, alkalicarbonates, alkalihydrogencarbonates, ammonia and organic, nitrogen containing bases, such as pyridine, triethylamine, triethanolamine.

9. The process according to one of claims 6 to 8, characterized in that after the addition of the corresponding alkaline earth metal salt solution the pH-value is adjusted to a value in the range from 8.5 to 9.5, especially 9.0, preferably by means of the addition of an aqueous ammonia solution.

10. The process according to one of claims 6 to 9, characterized in that the reaction is carried out at a temperature in the range from 10°C to 30°C, preferably at room temperature.

11. The process according to one of claims 6 to 10, characterized in that the magnesium salt of the N⁵,N¹⁰-methylene-5,6,7,8-tetrahydrofolic acid is prepared by reacting at room temperature the 5,6,7,8-tetrahydrofolic acid in water with a compound, selected from the group, consisting of alkalihydroxides, alkalicarbonates, alkalihydrogencarbonates, ammonia and organic, nitrogen containing bases, such as for example pyridine, triethylamine, triethanolamine, then reacting the so prepared salt solution with formaldehyde, and then adding an aqueous solution of a magnesium salt, whereby the magnesium salt of the N⁵,N¹⁰-methylene-5,6,7,8-tetrahydrofolic acid precipitates.

12. The process according to one of claims 6 to 10, characterized in that the calcium salt of the N⁵,N¹⁰-methylene-5,6,7,8-tetrahydrofolic acid is prepared by reacting at room temperature the 5,6,7,8-tetrahydrofolic acid in water with a compound, selected from the group, consisting of alkalihydroxides, alkalicarbonates, alkalihydrogencarbonates, ammonia and organic, nitrogen containing bases, such as for example pyridine, triethylamine, triethanolamine,
then adding to the so prepared salt solution a watersoluble, aromatic, nitrogen containing base, which is especially selected from the group, consisting of pyridine and substituted pyridines, for example picolines, lutidines, ethylpyridines, whereby pyridine is preferred,
then reacting the so prepared salt solution with formaldehyde, and then adding an aqueous solution of a calcium salt, whereby the calcium salt of the N⁵,N¹⁰-methylene-5,6,7,8-tetrahydrofolic acid precipitates.

13. Use of the salts, which are prepared according to claim 3, for the preparation of a medicament for the synergistic excertion of influence of a cancer controlling compound and/or for the reduction of the toxicity of a cancer controlling compound and/or for the protection of human and/or animal cells.

14. Use according to claim 13, characterized in that the salt is present per dosis unit in the medicament in an amount from 1 mg to 500 mg, especially from 5 mg to 150 mg.

15. Use according to one of claims 13 to 14, characterized in that the medicament is in the form of a parenteral and/or oral, pharmaceutical preparation.

## Revendications (Revendications pour l'(les) Etat(s) contractant(s) suivant(s): AT, BE, CH, DE, DK, FR, GB, IT, LI, LU, NL, SE)

1. Sels pharmaceutiquement et/ou thérapeutiquement acceptables de l'acide N⁵,N¹⁰-méthylène-5,6,7,8-tétrahydrofolique, répondant aux formules Ia et Ib 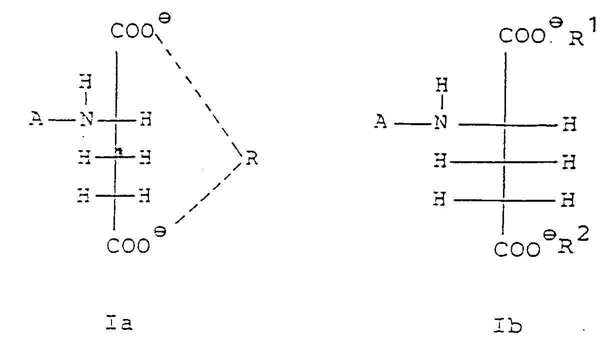 sous forme du mélange de diastéréo-isomères (6RS) ou sous forme des diastéréo-isomères individuels (6R) ou (6S), dans lesquelles
R représente un cation organique polyvalent ou encore Ca⁺ ou Mg⁺,
R¹ et R représentent un cation organique monovalent, R¹ et R pouvant être identiques ou différents, et
A représente un radical de formule

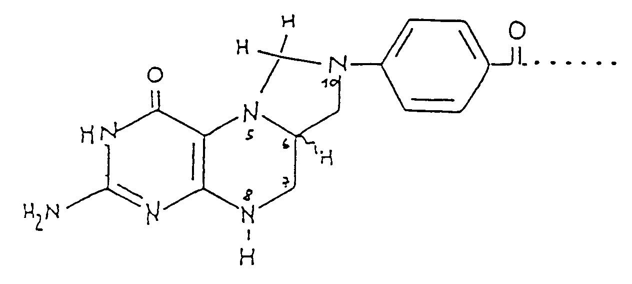

2. Sels selon la revendication 1, caractérisés en ce que les cations organiques sont choisis parmi le groupe constitué par l'éthanolamine, la triéthanolamine, le 2-diméthylaminoéthanol, la tert.-butylamine, un acide aminocarboxylique basique tel que par exemple la lysine, en particulier la (L)-lysine, ou l'arginine, en particulier la (L) -arginine.

3. Procédé pour la préparation de sels selon l'une quelconque des revendications 1 à 2, caractérisé en ce qu'on dépose au préalable l'acide N⁵,N¹⁰-méthylène-5,6,7,8-tétrahydrofolique de formule II 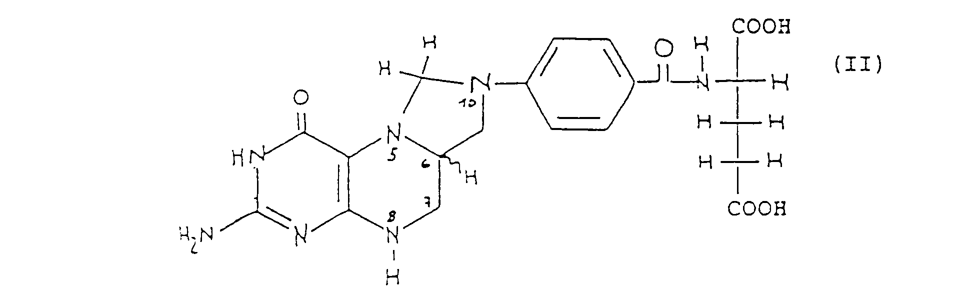 sous forme du mélange de diastéréo-isomères (6RS) ou sous forme des diastéréo-isomères individuels (6R) ou (6S), et on l'amène ensuite à réagir avec une base organique et/ou inorganique correspondante ou avec un sel de Ca⁺ ou de Mg⁺.

4. Procédé selon la revendication 3, caractérisé en ce que la réaction a lieu dans au moins un solvant, de préférence choisi parmi le groupe comprenant le diméthylsulfoxyde, le diméthylformamide, le N,N-diméthylacétamide, la N-méthylpyrrolidone et l'eau.

5. Procédé pour la préparation de sels selon l'une quelconque des revendications 1 à 2, caractérisé en ce qu'on prépare un sel de métal alcalino-terreux répondant à la formule III 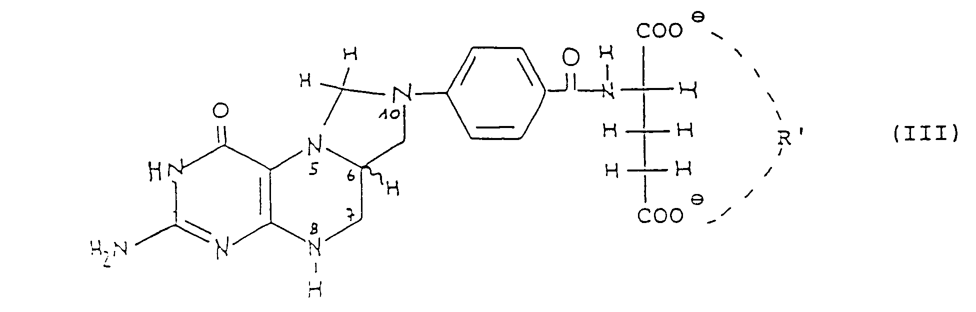 dans laquelle
R′ représente Ca⁺ ou Mg⁺,
conformément au procédé selon l'une quelconque des revendications 3 à 4, et on l'amène ensuite à réagir avec un sel correspondant de l'acide oxalique, dans lequel l'oxalate de métal alcalino-terreux précipite et dans lequel, simultanément, se forme le sel organique et/ou inorganique correspondant du composé de formule I, ensuite on sépare l'oxalate de métal alcalino-terreux précipité, puis on précipite hors de la solution le sel désiré de formule I par addition d'au moins un solvant organique miscible à l'eau, par exemple l'acétone, le méthanol ou l'éthanol.

6. Procédé pour la préparation de sels de métaux alcalino-terreux de l'acide N⁵,N¹⁰-méthylène-5,6,7,8-tétrahydrofolique de formule I′ 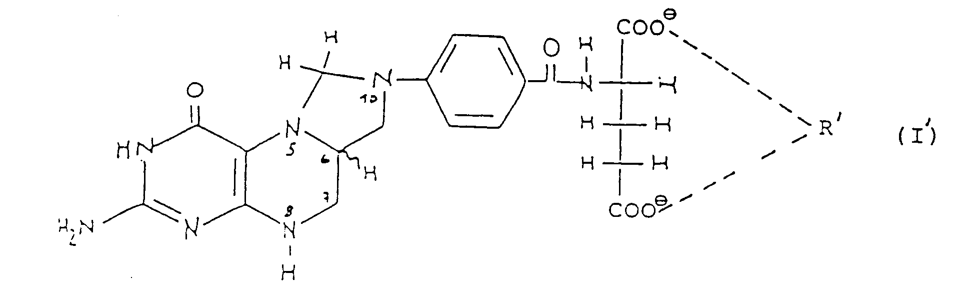 sous forme du mélange de diastéréo-isomères (6RS) ou bien sous forme des diastéréo-isomères individuels (6R) ou (6S), dans laquelle
R' représente Ca⁺ ou Mg⁺,
caractérisé en ce qu'on dépose au préalable une solution aqueuse d'un sel hydrosoluble de l'acide 5,6,7,8-tétrahydrofolique de formule IV 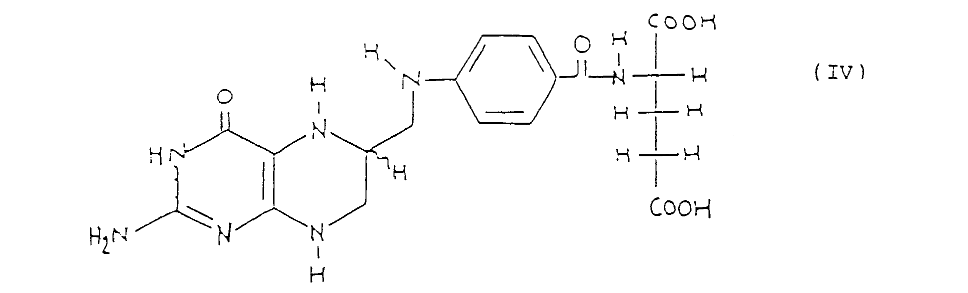 et on l'amène ensuite à réagir avec du formaldéhyde, puis on y ajoute une solution aqueuse d'un sel de Ca⁺ ou de Mg⁺, en sorte que le sel de métal alcalino-terreux correspondant de formule I′ précipite.

7. Procédé selon la revendication 6, caractérisé en ce que, avant l'addition du formaldéhyde, on ajoute encore une base azotée hydrosoluble, aromatique, que l'on choisit en particulier parmi le groupe constitué par la pyridine et par des pyridines substituées, par exemple les picolines, les lutidines, les éthylpyridines, la pyridine étant préférée.

8. Procédé selon l'une quelconque des revendications 6 à7, caractérisé en ce qu'on prépare le sel hydrosoluble de l'acide 5,6,7,8-tétrahydrofolique par mise en réaction de l'acide libre dans de l'eau avec un composé choisi parmi le groupe constitué par des hydroxydes de métaux alcalins, des carbonates de métaux alcalins, des hydrogénocarbonates de métaux alcalins, l'ammoniac et des bases azotées organiques telles que par exemple la pyridine, la triéthylamine, la triéthanolamine.

9. Procédé selon l'une quelconque des revendications 6 à8, caractérisé en ce que, après l'addition de la solution de sel de métal alcalino-terreux correspondante, on règle la valeur de pH à une valeur dans le domaine de 8,5 à 9,5, en particulier de 9,0, de préférence par addition d'une solution aqueuse d'ammoniac.

10. Procédé selon l'une quelconque des revendications 6 à9, caractérisé en ce qu'on effectue la réaction à une température dans le domaine de 10°C à 30°C, de préférence à la température ambiante.

11. Procédé selon l'une quelconque des revendications 6 à10, caractérisé en ce qu'on prépare le sel de magnésium de l'acide N⁵,N¹⁰-méthylène-5,6,7,8-tétrahydrofolique par mise en réaction, à la température ambiante, de l'acide 5,6,7,8-tétrahydrofolique dans de l'eau avec un composé choisi parmi le groupe constitué par des hydroxydes de métaux alcalins, des carbonates de métaux alcalins, des hydrogénocarbonates de métaux alcalins, l'ammoniac et des bases azotées organiques telles que par exemple la pyridine, la triéthylamine, la triéthanolamine; ensuite, on amène à réagir avec du formaldéhyde cette solution saline ainsi préparée, puis on y ajoute une solution aqueuse d'un sel de magnésium, en sorte que le sel de magnésium de l'acide N⁵,N¹⁰-méthylène-5,6,7,8-tétrahydrofolique précipite.

12. Procédé selon l'une quelconque des revendications 6 à10, caractérisé en ce qu'on prépare le sel de calcium de l'acide N⁵,N¹⁰-méthylène-5,6,7,8-tétrahydrofolique par mise en réaction, à la température ambiante, de l'acide 5,6,7,8-tétrahydrofolique dans de l'eau avec un composé choisi parmi le groupe constitué par des hydroxydes de métaux alcalins, des carbonates de métaux alcalins, des hydrogénocarbonates de métaux alcalins, l'ammoniac et des bases azotées organiques, par exemple la pyridine, la triéthylamine, la triéthanolamine,
puis on ajoute, à cette solution saline ainsi préparée, une base azotée aromatique, hydrosoluble, qui est choisie en particulier parmi le groupe constitué par la pyridine et par des pyridines substituées, par exemple les picolines, les lutidines, les éthylpyridines, la pyridine étant préférée,
ensuite, on amène à réagir avec du formaldéhyde cette solution saline ainsi préparée et on ajoute ensuite une solution aqueuse d'un sel de calcium, en sorte que le sel de calcium de l'acide N⁵,N¹⁰-méthylène-5,6,7,8-tétrahydrofolique précipite.

13. Médicament pour l'influence synergique d'un composé de lutte contre le cancer et/ou pour la diminution de la toxicité d'un composé de lutte contre le cancer et/ou pour la protection de cellules humaines et/ou animales, caractérisé en ce qu'il contient, à titre d'au moins un composé actif, au moins un sel selon l'une quelconque des revendications 1 à 2, de préférence en une quantité de 1 mg à 500 mg, en particulier de 5 mg à 150 mg par unité posologique, et en ce qu'il est présent de préférence sous la forme d'une préparation pharmaceutique à administrer par voie parentérale et/ou par voie orale.

14. Utilisation des sels selon l'une quelconque des revendications 1 à 2, pour la préparation d'un médicament pour l'influence synergique d'un composé de lutte contre le cancer et/ou pour la diminution de la toxicité d'un composé de lutte contre le cancer et/ou pour la protection de cellules humaines et/ou animales.

15. Utilisation selon la revendication 14, caractérisée en ce que le sel est présent dans le médicament, par unité posologique, en une quantité de 1 mg à 500 mg, en particulier de 5 mg à 150 mg.

16. Utilisation selon l'une quelconque des revendications 14 à 15, caractérisée en ce que le médicament est présent sous forme d'une préparation pharmaceutique à administration par voie parentérale et/ou par voie orale.

## Revendications (Revendications pour l'(les) Etat(s) contractant(s) suivant(s): ES)

1. Procédé pour la préparation de sels pharmaceutiquement et/ou thérapeutiquement acceptables de l'acide N⁵,N¹⁰-méthylène-5,6,7,8-tétrahydrofolique, répondant aux formules Ia et Ib 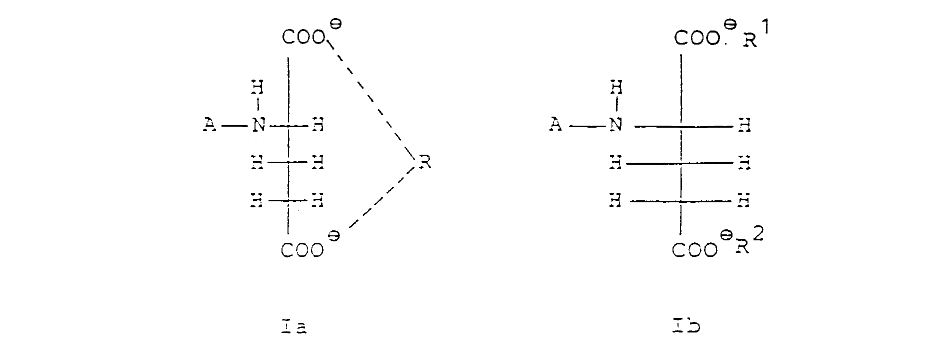 sous forme du mélange de diastéréo-isomères (6RS) ou sous forme des diastéréo-isomères individuels (6R) ou (6S), dans lesquelles
R représente un cation organique polyvalent ou encore Ca⁺ ou Mg⁺,
R¹ et R représentent un cation organique monovalent, R¹ et R pouvant être identiques ou différents, et
A représente un radical de formule 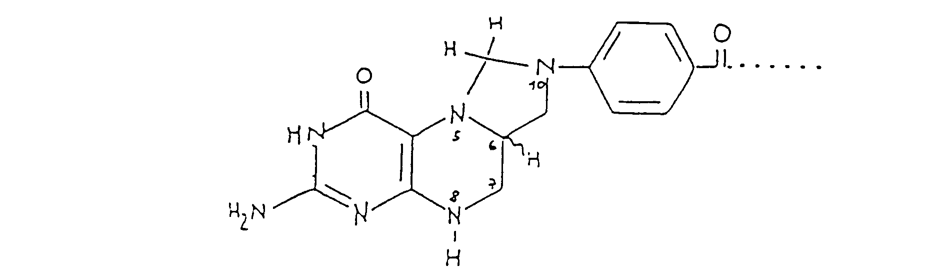
caractérisé en ce qu'on dépose au préalable l'acide N⁵,N¹⁰-méthylène-5,6,7,8-tétrahydrofolique de formule II 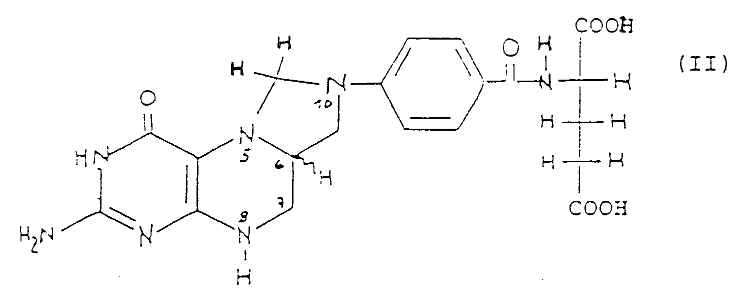 sous forme du mélange de diastéréo-isomères (6RS) ou sous forme des diastéréo-isomères individuels (6R) ou (6S), et on l'amène ensuite à réagir avec une base organique et/ou inorganique correspondante ou avec un sel de Ca⁺ ou de Mg⁺.

2. Procédé selon la revendication 1, caractérisé en ce que la réaction a lieu dans au moins un solvant, de préférence choisi parmi le groupe comprenant le diméthylsulfoxyde, le diméthylformamide, le N,N-diméthylacétamide, la N-méthylpyrrolidone et l'eau.

3. Procédé selon l'une quelconque des revendications 1 à2, caractérisé en ce qu'on choisit les cations organiques parmi le groupe constitué par l'éthanolamine, la triéthanolamine, le 2-diméthylaminoéthanol, la tert.-butylamine, un acide aminocarboxylique basique tel que par exemple la lysine, en particulier la (L)-lysine, ou l'arginine, en particulier la (L)-arginine.

4. Procédé pour la préparation de sels pharmaceutiquement et/ou thérapeutiquement acceptables de l'acide N⁵,N¹⁰-méthylène-5,6,7,8-tétrahydrofolique, répondant aux formules Ia et Ib 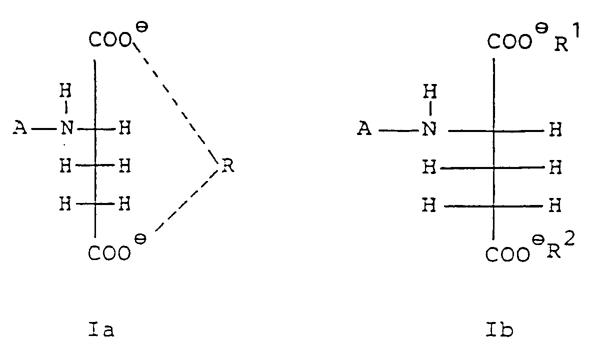 sous forme du mélange de diastéréo-isomères (6RS) ou sous forme des diastéréo-isomères individuels (6R) ou (6S), dans lesquelles
R représente un cation organique polyvalent ou encore Ca⁺ ou Mg⁺,
R¹ et R représentent un cation organique monovalent, R¹ et R pouvant être identiques ou différents, et
A représente un radical de formule 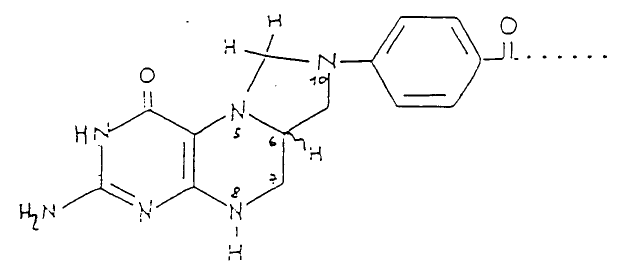
caractérisé en ce qu'on prépare un sel de métal alcalino-terreux répondant à la formule III 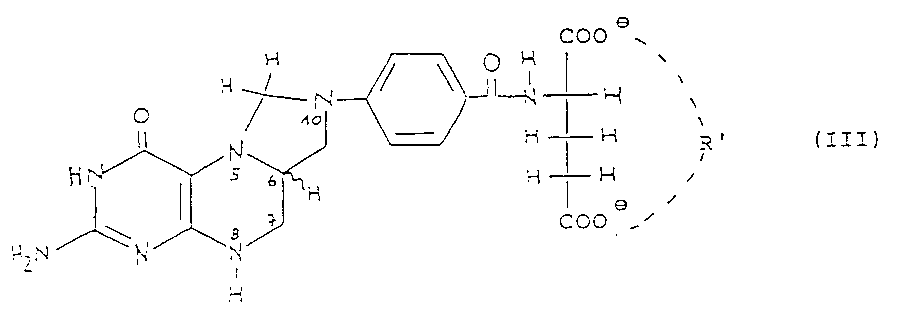 dans laquelle
R' représente Ca⁺ ou Mg⁺,
conformément au procédé selon l'une quelconque des revendications 1 à 3, et on l'amène ensuite à réagir avec un sel correspondant de l'acide oxalique, dans lequel l'oxalate de métal alcalino-terreux précipite et dans lequel, simultanément, se forme le sel organique et/ou inorganique correspondant du composé de formule I, ensuite on sépare l'oxalate de métal alcalino-terreux précipité, puis on précipite hors de la solution le sel désiré de formule I par addition d'au moins un solvant organique miscible à l'eau, par exemple l'acétone, le méthanol ou l'éthanol.

5. Procédé pour la préparation de sels de métaux alcalino-terreux de l'acide N⁵,N¹⁰-méthylène-5,6,7,8-tétrahydrofolique de formule I′ 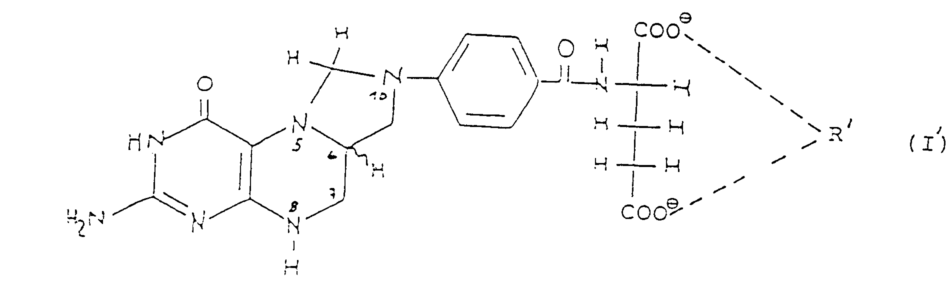 sous forme du mélange de diastéréo-isomères (6RS) ou bien sous forme des diastéréo-isomères individuels (6R) ou (6S), dans laquelle
R' représente Ca⁺ ou Mg⁺,
caractérisé en ce qu'on dépose au préalable une solution aqueuse d'un sel hydrosoluble de l'acide 5,6,7,8-tétrahydrofolique de formule IV 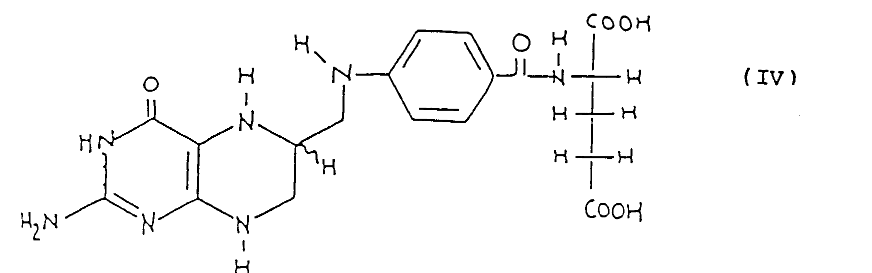 et on l'amène ensuite à réagir avec du formaldéhyde, puis on y ajoute une solution aqueuse d'un sel de Ca⁺ ou de Mg⁺, en sorte que le sel de métal alcalino-terreux correspondant de formule I′ précipite.

6. Procédé selon la revendication 5, caractérisé en ce que, avant l'addition du formaldéhyde, on ajoute encore une base azotée hydrosoluble, aromatique, que l'on choisit en particulier parmi le groupe constitué par la pyridine et par des pyridines substituées, par exemple les picolines, les lutidines, les éthylpyridines, la pyridine étant préférée.

7. Procédé selon l'une quelconque des revendications 5 à6, caractérisé en ce qu'on prépare le sel hydrosoluble de l'acide 5,6,7,8-tétrahydrofolique par mise en réaction de l'acide libre dans de l'eau avec un composé choisi parmi le groupe constitué par des hydroxydes de métaux alcalins, des carbonates de métaux alcalins, des hydrogénocarbonates de métaux alcalins, l'ammoniac et des bases azotées organiques telles que par exemple la pyridine, la triéthylamine, la triéthanolamine.

8. Procédé selon l'une quelconque des revendications 5 à7, caractérisé en ce que, après l'addition de la solution de sel de métal alcalino-terreux correspondante, on règle la valeur de pH à une valeur dans le domaine de 8,5 à 9,5, en particulier de 9,0, de préférence par addition d'une solution aqueuse d'ammoniac.

9. Procédé selon l'une quelconque des revendications 5 à8, caractérisé en ce qu'on effectue la réaction à une température dans le domaine de 10°C à 30°C, de préférence à la température ambiante.

10. Procédé selon l'une quelconque des revendications 5 à9, caractérisé en ce qu'on prépare le sel de magnésium de l'acide N⁵,N¹⁰-méthylène-5,6,7,8-tétrahydrofolique par mise en réaction, à la température ambiante, de l'acide 5,6,7,8-tétrahydrofolique dans de l'eau avec un composé choisi parmi le groupe constitué par des hydroxydes de métaux alcalins, des carbonates de métaux alcalins, des hydrogénocarbonates de métaux alcalins, l'ammoniac et des bases azotées organiques telles que par exemple la pyridine, la triéthylamine, la triéthanolamine; ensuite, on amène à réagir avec du formaldéhyde cette solution saline ainsi préparée, puis on y ajoute une solution aqueuse d'un sel de magnésium, en sorte que le sel de magnésium de l'acide N⁵,N¹⁰-méthylène-5,6,7,8-tétrahydrofolique précipite.

11. Procédé selon l'une quelconque des revendications 5 à9, caractérisé en ce qu'on prépare le sel de calcium de l'acide N⁵,N¹⁰-méthylène-5,6,7,8-tétrahydrofolique par mise en réaction, à la température ambiante, de l'acide 5,6,7,8-tétrahydrofolique dans de l'eau avec un composé choisi parmi le groupe constitué par des hydroxydes de métaux alcalins, des carbonates de métaux alcalins, des hydrogénocarbonates de métaux alcalins, l'ammoniac et des bases azotées organiques, par exemple la pyridine, la triéthylamine, la triéthanolamine,
puis on ajoute, à cette solution saline ainsi préparée, une base azotée aromatique, hydrosoluble, qui est choisie en particulier parmi le groupe constitué par la pyridine et par des pyridines substituées, par exemple les picolines, les lutidines, les éthylpyridines, la pyridine étant préférée,
ensuite, on amène à réagir avec du formaldéhyde cette solution saline ainsi préparée et on ajoute ensuite une solution aqueuse d'un sel de calcium, en sorte que le sel de calcium de l'acide N⁵,N¹⁰-méthylène-5,6,7,8-tétrahydrofolique précipite.

12. Procédé pour la préparation de médicaments pour l'influence synergique d'un composé de lutte contre le cancer et/ou pour la diminution de la toxicité d'un composé de lutte contre le cancer et/ou pour la protection de cellules humaines et/ou animales, caractérisé en ce que, dans le médicament, outre les adjuvants et les substances de support habituels, à titre d'au moins un composé actif, on incorpore par mélange au moins un sel selon les revendications précédentes, de préférence en une quantité de 1 mg à 500 mg, en particulier de 5 mg à 150 mg par unité posologique, dans lequel le médicament est présent sous la forme d'une préparation pharmaceutique à administrer par voie parentérale et/ou par voie orale, et est administré comme tel.

## Revendications (Revendications pour l'(les) Etat(s) contractant(s) suivant(s): GR)

1. Sels pharmaceutiquement et/ou thérapeutiquement acceptables de l'acide N⁵,N¹⁰-méthylène-5,6,7,8-tétrahydrofolique, répondant aux formules Ia et Ib 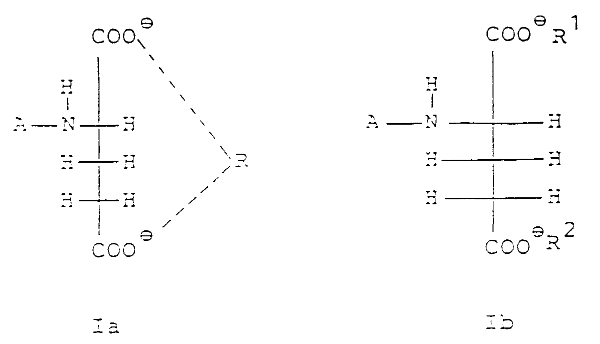 sous forme du mélange de diastéréo-isomères (6RS) ou sous forme des diastéréo-isomères individuels (6R) ou (6S), dans lesquelles
R représente un cation organique polyvalent ou encore Ca⁺ ou Mg⁺,
R¹ et R représentent un cation organique monovalent, R¹ et R pouvant être identiques ou différents, et
A représente un radical de formule 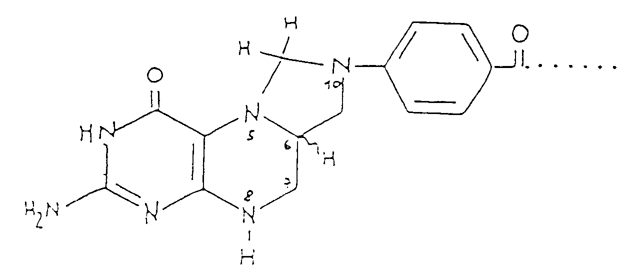
que l'on prépare conformément à la revendication 3.

2. Sels selon la revendication 1, caractérisés en ce que les cations organiques sont choisis parmi le groupe constitué par l'éthanolamine, la triéthanolamine, le 2-diméthylaminoéthanol, la tert.-butylamine, un acide aminocarboxylique basique tel que par exemple la lysine, en particulier la (L)-lysine, ou l'arginine, en particulier la (L)-arginine.

3. Procédé pour la préparation de sels selon l'une quelconque des revendications 1 à 2, caractérisé en ce qu'on dépose au préalable l'acide N⁵,N¹⁰-méthylène-5,6,7,8-tétrahydrofolique de formule II 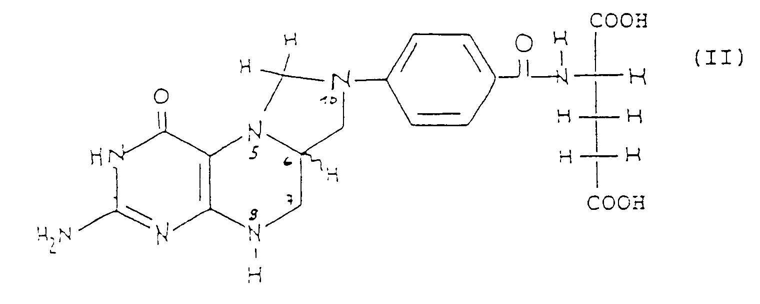 sous forme du mélange de diastéréo-isomères (6RS) ou sous forme des diastéréo-isomères individuels (6R) ou (6S), et on l'amène ensuite à réagir avec une base organique et/ou inorganique correspondante ou avec un sel de Ca⁺ ou de Mg⁺.

4. Procédé selon la revendication 3, caractérisé en ce que la réaction a lieu dans au moins un solvant, de préférence choisi parmi le groupe comprenant le diméthylsulfoxyde, le diméthylformamide, le N,N-diméthylacétamide, la N-méthylpyrrolidone et l'eau.

5. Procédé pour la préparation de sels selon l'une quelconque des revendications 1 à 2, caractérisé en ce qu'on prépare un sel de métal alcalino-terreux répondant à la formule III 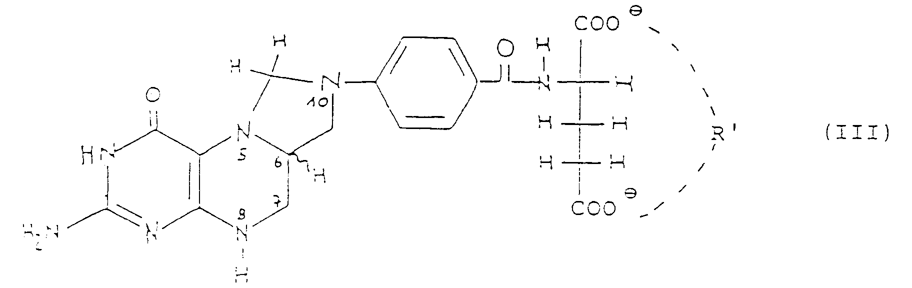 dans laquelle
R' représente Ca⁺ ou Mg⁺,
conformément au procédé selon l'une quelconque des revendications 3 à 4, et on l'amène ensuite à réagir avec un sel correspondant de l'acide oxalique, dans lequel l'oxalate de métal alcalino-terreux précipite et dans lequel, simultanément, se forme le sel organique et/ou inorganique correspondant du composé de formule I, ensuite on sépare l'oxalate de métal alcalino-terreux précipité, puis on précipite hors de la solution le sel désiré de formule I par addition d'au moins un solvant organique miscible à l'eau, par exemple l'acétone, le méthanol ou l'éthanol.

6. Procédé pour la préparation de sels de métaux alcalino-terreux de l'acide N⁵,N¹⁰-méthylène-5,6,7,8-tétrahydrofolique de formule I′ 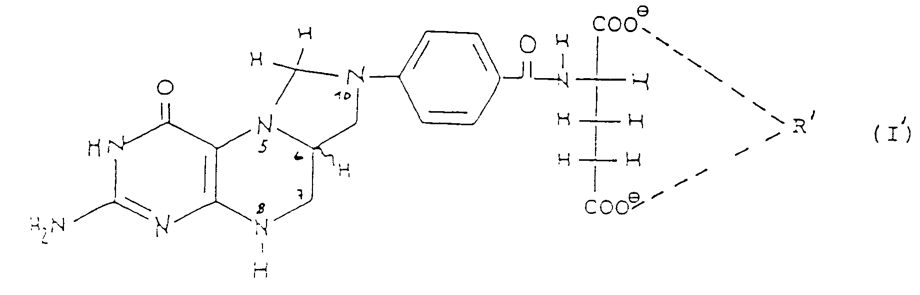 sous forme du mélange de diastéréo-isomères (6RS) ou bien sous forme des diastéréo-isomères individuels (6R) ou (6S), dans laquelle
R' représente Ca⁺ ou Mg⁺,
caractérisé en ce qu'on dépose au préalable une solution aqueuse d'un sel hydrosoluble de l'acide 5,6,7,8-tétrahydrofolique de formule IV 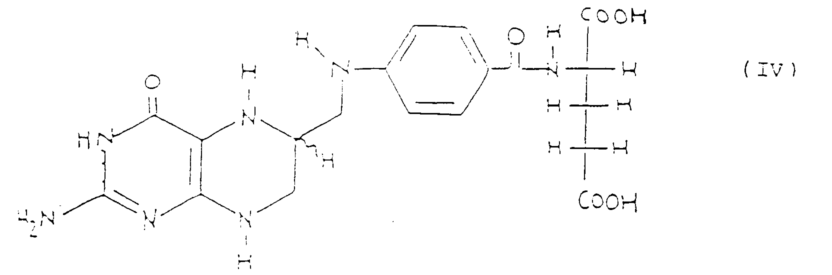 et on l'amène ensuite à réagir avec du formaldéhyde, puis on y ajoute une solution aqueuse d'un sel de Ca⁺ ou de Mg⁺, en sorte que le sel de métal alcalino-terreux correspondant de formule I′ précipite.

7. Procédé selon la revendication 6, caractérisé en ce que, avant l'addition du formaldéhyde, on ajoute encore une base azotée hydrosoluble, aromatique, que l'on choisit en particulier parmi le groupe constitué par la pyridine et par des pyridines substituées, par exemple les picolines, les lutidines, les éthylpyridines, la pyridine étant préférée.

8. Procédé selon l'une quelconque des revendications 6 à7, caractérisé en ce qu'on prépare le sel hydrosoluble de l'acide 5,6,7,8-tétrahydrofolique par mise en réaction de l'acide libre dans de l'eau avec un composé choisi parmi le groupe constitué par des hydroxydes de métaux alcalins, des carbonates de métaux alcalins, des hydrogénocarbonates de métaux alcalins, l'ammoniac et des bases azotées organiques telles que par exemple la pyridine, la triéthylamine, la triéthanolamine.

9. Procédé selon l'une quelconque des revendications 6 à8, caractérisé en ce que, après l'addition de la solution de sel de métal alcalino-terreux correspondante, on règle la valeur de pH à une valeur dans le domaine de 8,5 à 9,5, en particulier de 9,0, de préférence par addition d'une solution aqueuse d'ammoniac.

10. Procédé selon l'une quelconque des revendications 6 à9, caractérisé en ce qu'on effectue la réaction à une température dans le domaine de 10°C à 30°C, de préférence à la température ambiante.

11. Procédé selon l'une quelconque des revendications 6 à10, caractérisé en ce qu'on prépare le sel de magnésium de l'acide N⁵,N¹⁰-méthylène-5, 6,7,8-tétrahydrofolique par mise en réaction, à la température ambiante, de l'acide 5,6,7,8-tétrahydrofolique dans de l'eau avec un composé choisi parmi le groupe constitué par des hydroxydes de métaux alcalins, des carbonates de métaux alcalins, des hydrogénocarbonates de métaux alcalins, l'ammoniac et des bases azotées organiques telles que par exemple la pyridine, la triéthylamine, la triéthanolamine; ensuite, on amène à réagir avec du formaldéhyde cette solution saline ainsi préparée, puis on y ajoute une solution aqueuse d'un sel de magnésium, en sorte que le sel de magnésium de l'acide N⁵,N¹⁰-méthylène-5,6,7,8-tétrahydrofolique précipite.

12. Procédé selon l'une quelconque des revendications 6 à10, caractérisé en ce qu'on prépare le sel de calcium de l'acide N⁵,N¹⁰-méthylène-5,6,7,8-tétrahydrofolique par mise en réaction, à la température ambiante, de l'acide 5,6,7,8-tétrahydrofolique dans de l'eau avec un composé choisi parmi le groupe constitué par des hydroxydes de métaux alcalins, des carbonates de métaux alcalins, des hydrogénocarbonates de métaux alcalins, l'ammoniac et des bases azotées organiques, par exemple la pyridine, la triéthylamine, la triéthanolamine,
puis on ajoute, à cette solution saline ainsi préparée, une base azotée aromatique, hydrosoluble, qui est choisie en particulier parmi le groupe constitué par la pyridine et par des pyridines substituées, par exemple les picolines, les lutidines, les éthylpyridines, la pyridine étant préférée,
ensuite, on amène à réagir avec du formaldéhyde cette solution saline ainsi préparée et on ajoute ensuite une solution aqueuse d'un sel de calcium, en sorte que le sel de calcium de l'acide N⁵,N¹⁰-méthylène-5,6,7,8-tétrahydrofolique précipite.

13. Utilisation des sels qu'on prépare selon la revendication 3, pour la préparation d'un médicament pour l'influence synergique d'un composé de lutte contre le cancer et/ou pour la diminution de la toxicité d'un composé de lutte contre le cancer et/ou pour la protection de cellules humaines et/ou animales.

14. Utilisation selon la revendication 13, caractérisée en ce que le sel est présent dans le médicament, par unité posologique, en une quantité de 1 mg à 500 mg, en particulier de 5 mg à 150 mg.

15. Utilisation selon l'une quelconque des revendications 13 à 14, caractérisée en ce que le médicament est présent sous forme d'une préparation pharmaceutique à administration par voie parentérale et/ou par voie orale.
